# EUROPEAN PATENT SPECIFICATION

(11) **EP 2 810 066 B1**
(45) Date of publication and mention of the grant of the patent: **31.07.2019**
(21) Application number: 13741424.9
(22) Date of filing: 23.01.2013
(51) Int. Cl.: G01N 33/50

(54) **METHODS OF TREATMENT OF CANCER**
VERFAHREN ZUR BEHANDLUNG VON KREBS
MÉTHODES DE TRAITEMENT DU CANCER

(30) Priority: 24.01.2012 US 201261590131 P
(43) Date of publication of application: 10.12.2014
(73) Proprietor: Millennium Pharmaceuticals, Inc., Cambridge, MA 02139 (US)
(72) Inventor: BLAKEMORE, Stephen, J., Littleton, MA 01460 (US); DI BACCO, Alessandra, M., Newton, MA 02458 (US); MULLIGAN, George, J., Lexington, MA 02421 (US)
(74) Representative: Lau, Sarah Jane
(86) International application number: PCT/US2013/022765
(87) International publication number: WO 2013/112601

(56) References cited:
- WO-A1-2009/154737
- WO-A1-2013/112598
- US-A1- 2006 281 122
- US-A1- 2009 034 823
- US-A1- 2009 275 546
- US-A1- 2010 204 180
- GENIN E ET AL: "Proteasome Inhibitors: Recent Advances and New Perspectives in Medicinal Chemistry", CURRENT TOPICS IN MEDICINAL CHEMISTRY, BENTHAM SCIENCE PUBLISHERS LTD, HILVERSUM; NL, vol. 10, no. 3, 1 January 2010 (2010-01-01), pages 232-256, XP002725847, ISSN: 1568-0266, DOI: 10.2174/156802610790725515
- LISA J CRAWFORD ET AL: "Proteasome inhibitors in cancer therapy", JOURNAL OF CELL COMMUNICATION AND SIGNALING, SPRINGER NETHERLANDS, DORDRECHT, vol. 5, no. 2, 31 January 2011 (2011-01-31), pages 101-110, XP019899685, ISSN: 1873-961X, DOI: 10.1007/S12079-011-0121-7
- HU XIN ET AL: "[Study of the expression of nuclear factor-kappa B, matrix metalloproteinase-3, -9 on nasopharyngeal carcinoma and their clinical significance]", MEDLINE,, 1 August 2010 (2010-08-01), XP002743186,
- NAKANISHI C ET AL: "NUCLEAR FACTOR-KAPPAB INHIBITORS AS SENSITIZERS TO ANTICANCER DRUGS", NATURE REVIEWS. CANCER, NATUR PUBLISHING GROUP, LONDON, GB, vol. 5, no. 4, 1 April 2005 (2005-04-01), pages 297-309, XP009057803, ISSN: 1474-175X, DOI: 10.1038/NRC1588
- LI F ET AL: "Targeting transcription factor NF-@?B to overcome chemoresistance and radioresistance in cancer therapy", BBA - REVIEWS ON CANCER, ELSEVIER SCIENCE BV, AMSTERDAM, NL, vol. 1805, no. 2, 1 April 2010 (2010-04-01), pages 167-180, XP026953171, ISSN: 0304-419X [retrieved on 2010-01-14]
- KATHARINA AMSCHLER ET AL: "NF-[kappa]B Inhibition through Proteasome Inhibition or IKK[beta] Blockade Increases the Susceptibility of Melanoma Cells to Cytostatic Treatment through Distinct Pathways", JOURNAL OF INVESTIGATIVE DERMATOLOGY, vol. 130, no. 4, 26 November 2009 (2009-11-26), pages 1073-1086, XP55226349, US ISSN: 0022-202X, DOI: 10.1038/jid.2009.365
- BRUGNATELLI ET AL.: 'Weekly Administration of Gemcitabine Plus Docetaxel in Patients with Advanced Breast Cancer: A Phase 1 Study' ONCOLOGY 0030-2414 vol. 62, 01 January 2002, pages 33 - 38, XP008174448
- J. Duan ET AL: "Nuclear factor- B p65 small interfering RNA or proteasome inhibitor bortezomib sensitizes head and neck squamous cell carcinomas to classic histone deacetylase inhibitors and novel histone deacetylase inhibitor PXD101", MOLECULAR CANCER THERAPEUTICS, vol. 6, no. 1, 1 January 2007 (2007-01-01) , pages 37-50, XP55226347, US ISSN: 1535-7163, DOI: 10.1158/1535-7163.MCT-05-0285
- ERIK KUPPERMAN ET AL: "Evaluation of the Proteasome Inhibitor MLN9708 in Preclinical Models of Human Cancer", CANCER RESEARCH, AACR - AMERICAN ASSOCIATION FOR CANCER RESEARCH, US, vol. 70, no. 5, 3 January 2010 (2010-01-03), pages 1970-1980, XP002682750, ISSN: 0008-5472, DOI: 10.1158/0008-5472.CAN-09-2766 [retrieved on 2010-02-16]

## Description

### Field of the Invention

The present invention provides methods of treating cancer with a proteasome inhibitor, as set out in the claims. The present invention also provides a method for determining whether to treat a patient with cancer, as set out in the claims.

### Background of the Invention

Cancer is a cellular disorder characterized by uncontrolled or disregulated cell proliferation, decreased cellular differentiation, inappropriate ability to invade surrounding tissue, and/or ability to establish new growth at ectopic sites. Depending on the specific cancer involved, the treatment for cancer may involve surgery, radiotherapy, and chemotherapy. There remains a continuing need for new and improved treatments for patients with cancer.

Proteasome inhibition represents an important new strategy in cancer treatment. King et al., Science 274:1652-1659 (1996), describes an essential role for the ubiquitin-proteasome pathway in regulating cell cycle, neoplastic growth and metastasis. The authors teach that a number of key regulatory proteins, including cyclins, and the cyclin-dependent kinases p21 and p27^{KIP1}, are temporally degraded during the cell cycle by the ubiquitin-proteasome pathway. The ordered degradation of these proteins is required for the cell to progress through the cell cycle and to undergo mitosis.

The proteasome inhibitor VELCADE® (bortezomib; *N*-2-pyrazinecarbonyl-L-phenylalanine-L-leucineboronic acid) is the first proteasome inhibitor to achieve regulatory approval. Mitsiades et al., Current Drug Targets, 7:1341 (2006), reviews the clinical studies leading to the approval of bortezomib for the treatment of multiple myeloma patients who have received at least one prior therapy. Fisher et al., J. Clin. Oncol., 30:4867, describes an international multi-center Phase II study confirming the activity of bortezomib in patients with relapsed or refractory mantle cell lymphoma. Ishii et al., Anti-Cancer Agents in Medicinal Chemistry, 7:359 (2007), and Roccaro et al., Curr. Pharm. Biotech., 7:1341 (2006), discuss a number of molecular mechanisms that may contribute to the antitumor activities of bortezomib. The proteasome inhibitor MLN9708 [2,2'-{2-[(1*R* )-1-({[(2,5-dichlorobenzoyl)amino]acetyl}amino)-3-methylbutyl]-5-oxo-1,3,2-dioxaborolane-4,4-diyl}diacetic acid] is currently undergoing clinical evaluation for hematological and solid cancers. MLN9708 is a citrate ester which rapidly hydrolyzes to the active form [(1R)-1-({[(2,5-dichlorobenzoyl)amino]acetyl}amino)-3-methylbutyl]boronic acid (MLN2238) on exposure to aqueous solution or plasma. MLN9708 has demonstrated anti-tumor activity in a range of hematological and solid tumor xenograft models (Kupperman et al. (2010) Cancer Res. 70:1970-1980). There remains a further need to identify cancer patients most likely to benefit from treatment with a proteasome inhibitor.

### Summary

The invention relates to the discovery that patients with cancer respond to treatment with proteasome inhibitors. In one aspect, the invention relates to increased expression of Nuclear Factor Kappa-B RelA 65,000 dalton subunit (NFκB p65) in biological samples comprising cells obtained from patients with cancer and responsive to proteasome inhibitors. Accordingly, the invention features treating cancer patients with a proteasome inhibitor if a sample from the patient demonstrates an elevated expression of NFκB p65, as set out in the claims.

### Description of Figures

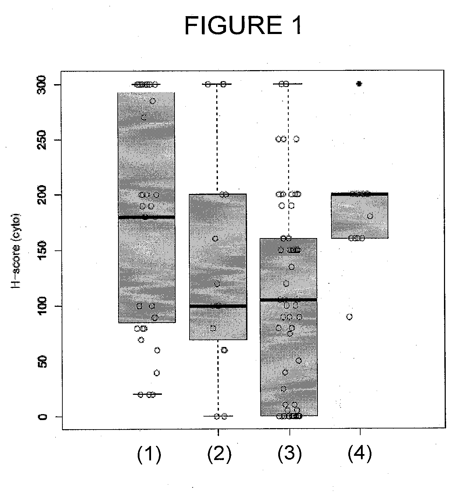

FIGURE 1 shows H-scores as measured by an NPκB p65 IHC assay in head and neck cancers as described in Example 2 below.

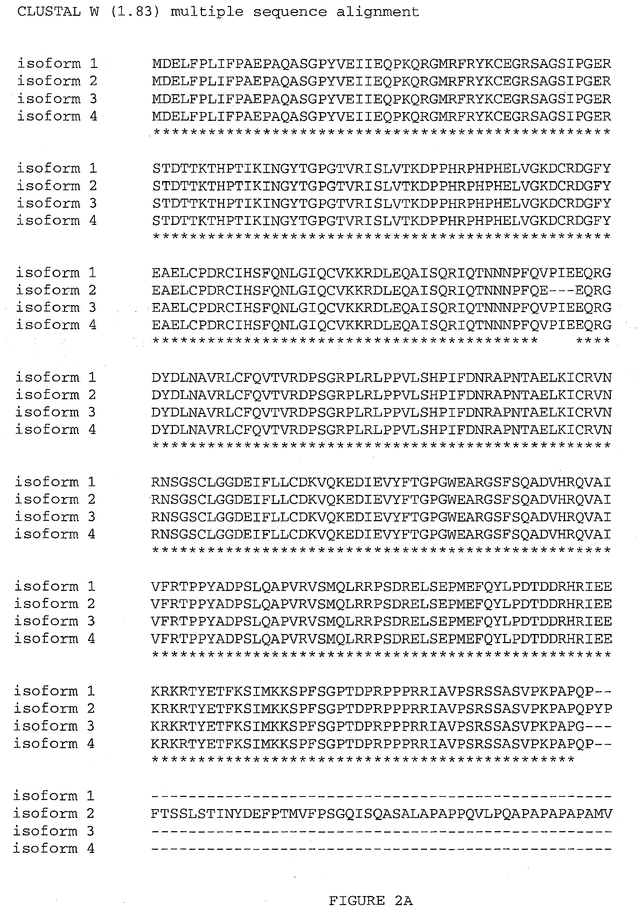

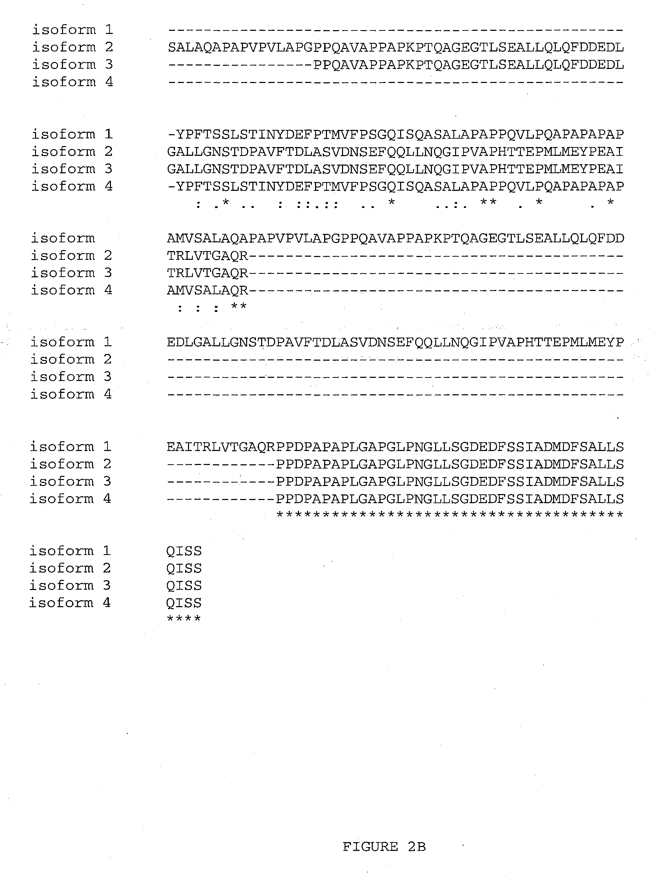

FIGURE 2. Figures 2A-2B show a multiple sequence alignment (Clustal W method) comparing the sequences of the isoforms of NFκB p65. NPκB p65 isoform 1 is SEQ ID NO:1, NFκB p65 isoform 2 is SEQ ID NO:2, NPκB p65 isoform 3 is SEQ ID NO:3 and NPκB p65 isoform 4 is SEQ ID NO:4. An asterisk (*) beneath a residue in the alignment indicates that the residue is the same in all four isoforms. A dash (-) at a position in a sequence in the alignment indicates that the alignment does not place a residue from that sequence in that position occupied by a residue shown for another isoform in the alignment.

### Description of the Invention

The present invention provides methods for treating cancer, as set out in the claims.

In another aspect, the present invention provides a method for determining whether to treat a patient with cancer with a proteasome inhibitor or a pharmaceutically acceptable salt or pharmaceutical composition thereof; as set out in the claims.

The term "alkyl", used alone or as part of a larger moiety, refers to a straight or branched chain or cyclic aliphatic group having from 1 to 12 carbon atoms. The term "alkoxy" refers to an -O-alkyl radical.

The terms "aryl" and "ar-", used alone or as part of a larger moiety, e.g., "aralkyl", "aralkoxy", or "aryloxyalkyl", refer to a C₆ to C14 aromatic hydrocarbon, comprising one to three rings, each of which is optionally substituted. Preferably, the aryl group is a C₆₋₁₀ aryl group. Aryl groups include, without limitation, phenyl, naphthyl, and anthracenyl. An "aralkyl" or "arylalkyl" group comprises an aryl group covalently attached to an alkyl group, either of which independently is optionally substituted. Preferably, the aralkyl group is C₆₋₁₀ aryl(C₁₋₆)alkyl, C₆₋₁₀ aryl(C₁₋₄)alkyl, or C₆₋₁₀ aryl(C₁₋₃)alkyl, including, without limitation, benzyl, phenethyl, and naphthylmethyl.

The term "substituted", as used herein, means that a hydrogen radical of the designated moiety is replaced with the radical of a specified substituent, provided that the substitution results in a stable or chemically feasible compound. Nonlimiting examples of suitable substituents include C₁₋₆ alkyl, C₃₋₈ cycloalkyl, C₁₋₆alkyl(C₃₋₈)cycloalkyl, C₂₋₈ alkenyl, C₂₋₈ alkynyl, cyano, amino, C₁₋₆ alkylamino, di(C₁₋₆)alkylamino, benzylamino, dibenzylamino, nitro, carboxy, carbo(C₁₋₆)alkoxy, trifluoromethyl, halogen, C₁₋₆ alkoxy, C₆₋₁₀ aryl, C₆₋₁₀ aryl(C₁₋₆)alkyl, C₆₋₁₀ aryl(C₁₋₆)alkoxy, hydroxy, C₁₋₆ alkylthio, C₁₋₆ alkylsulfinyl, C₁₋₆ alkylsulfonyl, C₆₋₁₀ arylthio, C₆₋₁₀ arylsulfinyl, C₆₋₁₀ arylsulfonyl, C₆₋₁₀ aryl, C₁₋₆ alkyl(C₆₋₁₀)aryl, and halo(C₆₋₁₀)aryl.

The phrase "one or more substituents", as used herein, refers to a number of substituents that equals from one to the maximum number of substituents possible based on the number of available bonding sites, provided that the above conditions of stability and chemical feasibility are met. Unless otherwise indicated, an optionally substituted group may have a substituent at each substitutable position of the group, and the substituents may be either the same or different. As used herein, the term "independently selected" means that the same or different values may be selected for multiple instances of a given variable in a single compound.

Unless otherwise explicitly stated, the term "proteasome" is intended to refer to constitutive proteasome, immunoproteasome, or both.

The term "about" is used herein to mean approximately, in the region of, roughly, or around. When the term "about" is used in conjunction with a numerical range, it modifies that range by extending the boundaries above and below the numerical values set forth. In general, the term "about" is used herein to modify a numerical value above and below the stated value by a variance of 10%.

As used herein, the term "comprises" means "includes, but is not limited to."

As used herein, the term "patient", means an animal, preferably a mammal, more preferably a human.

As used herein, the term "cancer" refers to a cellular disorder characterized by uncontrolled or disregulated cell proliferation, decreased cellular differentiation, inappropriate ability to invade surrounding tissue, and/or ability to establish new growth at ectopic sites. The term "cancer" further encompasses primary and metastatic cancers.

As used herein, the term "therapeutically effective amount" means an amount that is sufficient upon appropriate administration to a patient (a) to cause a detectable decrease in the severity of the disorder or disease state being treated; (b) to ameliorate or alleviate the patient's symptoms of the disease or disorder; or (c) to slow or prevent advancement of, or otherwise stabilize or prolong stabilization of, the disorder or disease state being treated (*e.g*., prevent additional tumor growth of a cancer). It should also be understood that a specific dosage and treatment regimen for any particular patient will depend upon a variety of factors, including the activity of the specific compound employed, the age, body weight, general health, sex, and diet of the patient, time of administration, rate of excretion, drug combinations, the judgment of the treating physician, and the severity of the particular disease being treated.

As used herein, the term "treating cancer" means treating a patient having, or at risk of developing or experiencing a recurrence of, cancer.

As used herein, the term "IHC" means immunohistochemistry. IHC refers to the process of detecting antigens (e.g., proteins) in cells of a tissue section by exploiting the principle of antibodies binding specifically to antigens in biological tissues.

As used herein the term "NFκB p65 IHC assay" or "Nuclear Factor Kappa-B p65 immunohistochemistry assay" refers to an IHC assay that measures the amount of the NPκB p65 protein in a tissue section of a tumor sample by using a antibody or antigen-binding fragment thereof (Fv, Fab, scFv, Fab' or F(ab')) that binds to p65 (NFKB3, GenPept Accession No. NP_068810 or an isoform thereof; product of RELA gene, ID 5970; sequences associated with p65 and its isoforms can be found at the website maintained by the National Institute for Biotechnology Information, Bethesda, MD.). p65 antibodies or antibody fragments thereof can be prepared by one of skill in the art or purchased commercially. In some embodiments, the p65 antibody is a rabbit monoclonal antibody. In some embodiments, the p65 antibody is a rabbit polyclonal antibody. In some embodiments, the p65 antibody is a goat polyclonal antibody. The NPκB p65 IHC assay can be an *in vitro* assay.

Antibodies which bind to NFκB p65 (anti-NFκB p65 antibodies) are readily available from commercial sources; for example, from Cell Signaling Technology, Danvers, MA; Invitrogen Corporation, Camarillo CA; Santa Cruz Biotechnology, Inc., Santa Cruz, CA; Abcam, Cambridge, MA; Rockland Immunochemicals, Inc., Gilbertsville, PA, Novus Biologicals, Littleton, CO; or BD Biosciences, San Jose, CA. Alternatively, an antibody which binds NFκB p65 can be generated by any of a number of methods known to those skilled in the art; such as by immunizing an animal, such as a chicken, mouse, rat, dog, sheep, cow, goat or rabbit with NFκB p65 or a portion thereof, such as a peptide isolated or cleaved and purified from the whole protein, expressed recombinantly as a partial length protein, or synthesized chemically. In some embodiments, the animal is a rabbit. In some embodiments, the animal is a goat. Further details on how to generate and isolate antibodies can be found in reference texts, such as Antibodies: A Laboratory Manual (1988) Harlow and Lane, Cold Spring Harbor Laboratory Press, Cold Spring Harbor, NY.

As used herein the term "antibody" is used in the broadest sense and specifically covers full length monoclonal antibodies, immunoglobulins, polyclonal antibodies, multispecific antibodies (e.g. bispecific antibodies) formed from at least two full length antibodies, e.g., each to a different antigen or epitope, and individual antigen binding fragments, including dAbs, scFv, Fab, F(ab)'₂, Fab', including human, humanized and antibodies from non-human species and recombinant antigen binding forms such as monobodies and diabodies.

Development, i.e., processing for visualization of antibody binding, of NFκB p65 IHC assay and measurement of NFκB p65 amount can be by a variety of methods known in the art. Development and quantification of antibody binding to NFκB p65 can be through a detectable label. Labeling can be direct or indirect. The label can be colorimetric, fluorescent or radioactive. In some embodiments, an antibody which binds to NFκB p65 directly comprises a detectable label. In other embodiments, development is through a reagent, such as a secondary antibody or biotin-avidin complex, which comprises a label and binds to the NFκB p65-binding antibody.

As used herein the term "H-score" is used to mean an immunohistology score for a tumor sample. In an attempt to accurately describe the extent of immunohistochemical staining of a tumor, the degree of IHC staining, if any, in each sub-cellular compartment in tumor cells is captured for each analyte. This algorithm includes capturing the percentage of tumor cells stained at each intensity level. A semi-quantitative intensity scale ranging from 0 for no staining to 3+ for the most intense staining is used. All of this information can be analyzed separately or used to calculate a variable, more continuous than simply Positive versus Negative, called the H-Score. This score is more representative of the staining of the entire tumor on the section. Although given sections may share the same simple intensity score, there is a difference between a 3+ case with only 10% of the cells staining as compared to a 3+ case where greater than 90% of the cells are staining. This difference is easily picked up using H-Score method. An H-Score is typically calculated for staining of each sub-cellular compartment for both normal and tumor cells using the following formula; H-Score = (% cells at 0) ^{∗} 0 + (% cells at 1+) ^{∗} 1 + (% cells at 2+) ^{∗} 2 + (% cells at 3+) ^{∗} 3. Thus, this score produces a continuous variable that ranges from 0 to 300.

For IHC assays, controls may include paraffin section of cell blocks prepared from admixtures of target positive cells and non-target cells (Liu et al. (2002) Am. J. Clin Pathol. 118:216), such as mixtures of cell lines with positive NPκB p65 amounts and peripheral blood leukocytes. These cell blocks can contain mixtures of target cells at the 10%, 30%, 50%, 80%, and 90% levels.

Other controls for assays to measure elevated NPκB p65 amount, such as an NFκB p65 IHC assay, can be a sample of non-cancerous tissue. In one embodiment, a sample of non-cancerous tissue can be tissue obtained in the vicinity of the tumor from the same patient from whom the tumor sample is obtained, either in the same tissue specimen or a separate tissue specimen. In another embodiment, a sample of non-cancerous tissue can be tissue from an animal, such as a human, without cancer. Controls for assay performance can include tissues with known high or low amounts of NFκB p65 expression. For example, tissues with B-cell maturation centers, such as tonsils, can have high levels of NFκB p65 expression and can serve as a positive control. Conversely, less active tissues can serve as a negative control. A suitable negative control tissue can be a non-cancerous adult liver sample.

In some embodiments, the H-score is determined manually by a trained pathologist. In some embodiments, the H-score is determined using an automated cellular imaging sytem and software. In some embodiments, the H-score is determined using a trained pathologist supplemented by an automated scoring method. See for example Choudhury et al., J. Histochem. Cytochem. 58: 95-107 (2010).

A H-score may be given a numerical value between 1 and 300 or may be expressed using values such as low, medium and high. As used herein a low H-score is defined as between 10 and 100; a medium H-score is defined as between 101 and 200; and a high H-score is defined as between 201 and 300.

The level of NFκB p65 in a tumor sample may also be measured by using an anti-NFκB p65 antibody with western blotting and ELISA techniques. For such methods, a lysate is made from a homogenate of tissue or tumor sample. Differential lysis and isolation techniques can separate nuclei from cytoplasm to allow for quantification of nuclear NFκB p65 separately from quantification of cytoplasmic NFκB p65. Homogenates of cells or tissues lysed for such measurements can have appropriate inhibitors, such as protease inhibitors, known in the art to preserve protein structures against degradation. NFκB p65 levels can be quantified on western blots by using software to calculate NFκB p65 band intensity. Some ELISA assays can include the NF-κB DNA response element bound by NFκB p65 to capture NFκB p65 from the lysate for subsequent detection and quantification with the anti-NFκB p65 antibody.

The level of NFκB p65 expression in a tumor sample may also be measured by immunofluorescence, such as of anti-NFκB p65 antibody bound to a tissue section, such as a frozen tissue section or to permeabilized isolated cells.

The level of NFκB p65 expression in a tumor sample may also be measured by RT-PCR using a probe that detects and/or primers that amplify the NFκB p65 mRNA (GenBank NM_021975 or variant thereof, e.g., a nucleic acid encoding any of the sequences selected from the group consisting of SEQ ID NO:1, SEQ ID NO:2, SEQ ID NO:3, SEQ ID NO:4). RNA can be obtained from either fresh tissue or tumor sample or from paraffin-embedded sections of tumor sample by methods known to those skilled in the art. Expression of NFκB p65 may also be inferred from an analysis of genes targeted with the NFκB response element. This analysis can further include quantification of genes such as NFKB1A and CCND2. See Weichert et al., B. J. Cancer 97:523-530 (2007).

Unless otherwise stated, structures depicted herein are also meant to include compounds which differ only in the presence of one or more isotopically enriched atoms. For example, compounds having the present structure except for the replacement of a hydrogen atom by a deuterium or tritium, or the replacement of a carbon atom by a ¹³C- or ¹⁴C-enriched carbon are within the scope of the invention.

As used herein, the term "proteasome inhibitor" refers to any substance which directly inhibits enzymatic activity of the 20S or 26S proteasome *in vitro* or *in vivo.* Proteasome inhibitors, their pharmacological properties and use in treating disease, including oncological diseases and inflammatory diseases are reviewed in Ruggeri et al. (2009) Adv. Pharmacol. 57:91-135. In some embodiments, the proteasome inhibitor is a peptidyl boronic acid. Examples of peptidyl boronic acid proteasome inhibitors suitable for use in the methods of the invention are disclosed in Adams et al., U.S. Patent Nos. 5,780,454 (1998), 6,066,730 (2000), 6,083,903 (2000); 6,297,217 (2001), 6,465,433 (2002), 6,548,668 (2003), 6,617,317 (2003), and 6,747,150 (2004), each of which is hereby incorporated by reference in its entirety, including all compounds and formulae disclosed therein. In some embodiments, the peptidyl boronic acid proteasome inhibitor is selected from the group consisting of: *N*-(4 morpholine)carbonyl-β-(1-naphthyl)-L-alanine-L-leucine boronic acid; *N*-(8 quinoline)sulfonyl- β-(1-naphthyl)-L-alanine-L-alanine-L-leucine boronic acid; *N*-(pyrazine)carbonyl-L-phenylalanine-L-leucine boronic acid, and *N*-(4 morpholine) carbonyl-[O-(2-pyridylmethyl)]-L-tyrosine-L-leucine boronic acid. In some embodiments, the proteasome inhibitor is N-(pyrazine)carbonyl-L-phenylalanine-L-leucine boronic acid (bortezomib; VELCADE®; formerly known as MLN341 or PS-341). Further examples of peptidyl boronic acid proteasome inhibitors are disclosed in Olhava and Danca, U.S. Patent Nos. 7,442,830, 7,867,662, and 8,003,819 each of which are herein incorporated by reference in their entirety, including all compounds and formulae disclosed therein. In some embodiments, the peptide boronic acid is disclosed in U.S. Patent No. 7,915,236, for example [(1R)-1-[[(2S,3R)-3-hydroxy-2-[(6-phenyl-pyridine-2-carbonyl)amino]-1-oxo-butyl]amino]-3-methylbutyl] boronic acid. (CEP-18870).

Further examples of peptidyl boronic acid proteasome inhibitors are disclosed in Fleming and Li, International Patent Publications WO 2010/036357 and WO 2011/123502.

Additionally, proteasome inhibitors include peptide aldehyde proteasome inhibitors (Stein et al., U.S. Patent No. 5,693,617 (1997); Siman et al., International Patent Publication WO 91/13904; Iqbal et al., J. Med. Chem. 38:2276-2277 (1995); and Iinuma et al., International Patent Publication WO 05/105826, each of which is hereby incorporated by reference in its entirety), peptidyl epoxy ketone proteasome inhibitors (Crews et al., U.S. Patent No. 6,831,099; Smyth et al., International Patent Publication WO 05/111008; Bennett et al., International Patent Publication WO 06/045066 or U.S. Patent Application Publication No. US20050245435, e.g., (*S*)-4-Methyl-*N*-((*S*)-1-(((*S*)-4-methyl-1-((*R*)-2-methyloxiran-2-yl)-1-oxopentan-2-yl)amino)-1-oxo-3-phenylpropan-2-yl)-2-((*S*)-2-(2-morpholinoacetamido)-4-phenylbutanamido)pentanamide (carfilzomib); U.S. Patent No. 7,687,456 e.g. L-Serinamide, O-methyl-N-[(2-methyl-5-thiazolyl)carbonyl]-L-seryl-O-methyl-N-[(1S)-2-[(2R)-2-methyl-2-oxiranyl]-2-oxo-1-(phenylmethyl)ethyl]-(ONX-0912); Spaltenstein et al. Tetrahedron Lett. 37:1343 (1996); Meng, Proc. Natl. Acad. Sci. 96: 10403 (1999); and Meng, Cancer Res. 59: 2798 (1999)), alpha-ketoamide proteasome inhibitors (Chatterjee and Mallamo, U.S. Patent Nos. 6,310,057 (2001) and 6,096,778 (2000); and Wang et al., U.S. Patent Nos. 6,075,150 (2000) and 6,781,000 (2004)), peptidyl vinyl ester proteasome inhibitors (Marastoni et al., J. Med. Chem. 48:5038 (2005), and peptidyl vinyl sulfone and 2-keto-1,3,4-oxadiazole proteasome inhibitors, such as those disclosed in Rydzewski et al., J. Med. Chem. 49:2953 (2006); and Bogyo et al., Proc. Natl. Acad. Sci. 94:6629 (1997)), azapeptoids and (Bouget et al., Bioorg. Med. Chem. 11:4881 (2003); Baudy-Floc'h et al., International Patent Publication WO 05/030707; and Bonnemains et al., International Patent Publication WO 03/018557), efrapeptin oligopeptides (Papathanassiu, International Patent Publication WO 05/115431), lactacystin and salinosporamide and analogs thereof (Fenteany et al., U.S. Patent Nos. 5,756,764 (1998), 6,147,223 (2000), 6,335,358 (2002), and 6,645,999 (2003); Fenteany et al., Proc. Natl. Acad. Sci. USA (1994) 91:3358; Fenical et al., International Patent Publication WO 05/003137; Palladino et al., International Patent Publication WO 05/002572; Stadler et al., International Patent Publication WO 04/071382; Xiao and Patel, U.S. Patent Application Publication No. 2005/023162; and Corey, International Patent Publication WO 05/099687).

In some embodiments, the proteasome inhibitor is bortezomib. In some embodiments, the proteasome inhibitor is selected from the group consisting of bortezomib, carfilizomib, ONX-0912, and CEP-18870. In some embodiments, the proteasome inhibitor is selected from the group consisting of carfilizomib, ONX-0912, and CEP-18870.

In some embodiments, proteasome inhibitor is characterized by a compound of formula (***I***): 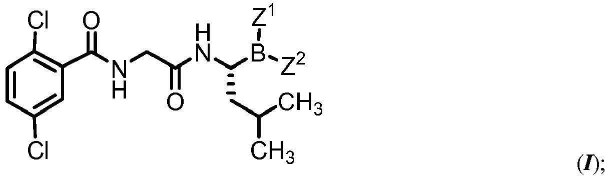 or a pharmaceutically acceptable salt or a pharmaceutical composition or a boronic acid anhydride thereof, wherein:
Z¹ and Z² are each independently hydroxy, alkoxy, aryloxy, or aralkoxy; or Z¹ and Z² together form a moiety derived from a boronic acid complexing agent.

As used herein, the term "boronic acid" refers to a chemical compound containing a -B(OH)₂ moiety. In some embodiments, boronic acid compounds can form oligomeric anhydrides by dehydration of the boronic acid moiety. For example, Snyder et al., J. Am. Chem. Soc. 80:3611 (1958), reports oligomeric arylboronic acids.

As used herein, the term "boronic acid anhydride" refers to a chemical compound formed by combination of two or more molecules of a boronic acid compound, with loss of one or more water molecules. When mixed with water, the boronic acid anhydride compound is hydrated to release the free boronic acid compound. In various embodiments, the boronic acid anhydride can comprise two, three, four, or more boronic acid units, and can have a cyclic or linear configuration. Non-limiting examples of oligomeric boronic acid anhydrides of peptide boronic acids compound of the invention are illustrated below:

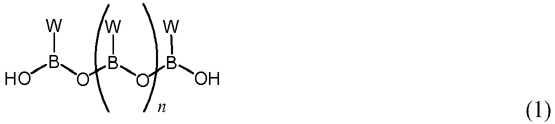

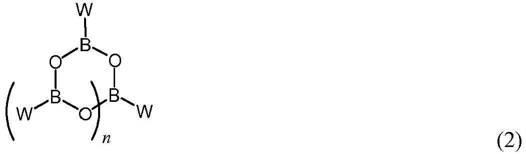

In formulae (1) and (2) directly above, the variable *n* is an integer from 0 to about 10, preferably 0, 1, 2, 3, or 4. In some embodiments, the boronic acid anhydride compound comprises a cyclic trimer ("boroxine") of formula (2), wherein *n* is 1. The variable W has the formula (3):

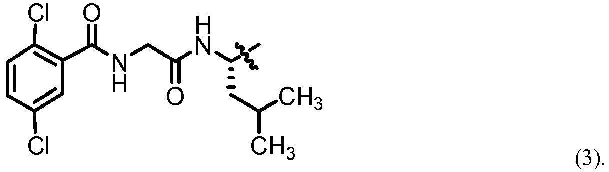

In some embodiments, at least 80% of the boronic acid present in the boronic acid anhydride compound exists in a single oligomeric anhydride form. In some embodiments, at least 85%, 90%, 95%, or 99% of the boronic acid present in the boronic acid anhydride compound exists in a single oligomeric anhydride form. In certain preferred embodiments, the boronic acid anhydride compound consists of, or consists essentially of, a boroxine having formula (3).

The boronic acid anhydride compound preferably can be prepared from the corresponding boronic acid by exposure to dehydrating conditions, including, but not limited to, recrystallization, lyophilization, exposure to heat, and/or exposure to a drying agent. Nonlimiting examples of suitable recrystallization solvents include ethyl acetate, dichloromethane, hexanes, ether, acetonitrile, ethanol, and mixtures thereof.

In some embodiments, Z¹ and Z² together form a moiety derived from a boronic acid complexing agent as disclosed in Olhava and Danca, U.S. Patent Nos. 7,442,830, 7,867,662, and 8,003,819 all of which are herein incorporated by reference in their entirety. For purposes of the invention, the term "boronic acid complexing agent" refers to any compound having at least two functional groups, each of which can form a covalent bond with boron. Nonlimiting examples of suitable functional groups include amino, hydroxyl, and carboxyl. In some embodiments, at least one of the functional groups is a hydroxyl group. The term "moiety derived from a boronic acid complexing agent" refers to a moiety formed by removing the hydrogen atoms from two functional groups of a boronic acid complexing agent.

As used herein, the terms "boronate ester" and "boronic ester" are used interchangeably and refer to a chemical compound containing a -B(Z¹)(Z²) moiety, wherein at least one of Z¹ or Z² is alkoxy, aralkoxy, or aryloxy; or Z¹ and Z² together form a moiety derived from a boronic acid complexing agent having at least one hydroxyl group.

In some embodiments, Z¹ and Z² are each hydroxy and the compound of formula (***I***) is characterized by formula (***II***): 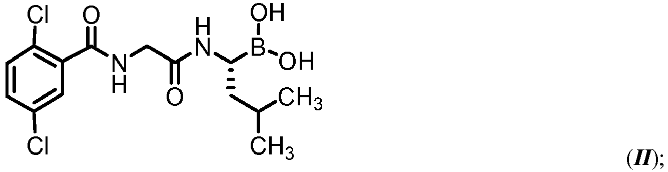 or a pharmaceutically acceptable salt or a pharmaceutical composition or a boronic acid anhydride thereof.

The compound of formula (***II***), [(1R)-1-({[(2,5-dichlorobenzoyl)amino]acetyl}amino)-3-methylbutyl]boronic acid (MLN2238) is disclosed in Olhava and Danca, U.S. Patent No. 7,442,830, herein incorporated by reference in its entirety.

In some other embodiments, Z¹ and Z² together form a moiety derived from a compound having at least two hydroxyl groups separated by at least two connecting atoms in a chain or ring, said chain or ring comprising carbon atoms and, optionally, a heteroatom or heteroatoms which can be N, S, or O, wherein the atom attached to boron in each case is an oxygen atom.

As employed herein, the term "compound having at least two hydroxyl groups" refers to any compound having two or more hydroxyl groups. For purposes of the invention, the two hydroxyl groups preferably are separated by at least two connecting atoms, preferably from about 2 to about 5 connecting atoms, more preferably 2 or 3 connecting atoms. For convenience, the term "dihydroxy compound" may be used to refer to a compound having at least two hydroxyl groups, as defined above. Thus, as employed herein, the term "dihydroxy compound" is not intended to be limited to compounds having only two hydroxyl groups. The moiety derived from a compound having at least two hydroxyl groups may be attached to boron by the oxygen atoms of any two of its hydroxyl groups. Preferably, the boron atom, the oxygen atoms attached to boron, and the atoms connecting the two oxygen atoms together form a 5- or 6-membered ring.

For purposes of the present invention, the boronic acid complexing agent preferably is pharmaceutically acceptable, i.e., suitable for administration to humans. In some preferred embodiments, the boronic acid complexing agent is a sugar, as described, e.g., in Plamondon et al., WO 02/059131 and Gupta et al., WO 02/059130. The term "sugar" includes any polyhydroxy carbohydrate moiety, including monosaccharides, disaccharides, polysaccharides, sugar alcohols and amino sugars. In some embodiments, the sugar is a monosaccharide, disaccharide, sugar alcohol, or amino sugar. Non-limiting examples of suitable sugars include glucose, sucrose, fructose, trehalose, mannitol, sorbitol, glucosamine, and *N*-methylglucosamine. In certain embodiments, the sugar is mannitol or sorbitol. Thus, in the embodiments wherein the sugar is mannitol or sorbitol, Z¹ and Z² together form a moiety of formula C₆H₁₂O₆, wherein the oxygen atoms of the two deprotonated hydroxyl groups form covalent attachments with boron to form a boronate ester compound. In certain embodiments, Z¹ and Z² together form a moiety derived from D-mannitol as disclosed in U.S. Patent Nos. 7,442,830.

In some embodiments, the boronic acid complexing agent is an alpha-hydroxycarboxylic acid or a beta-hydroxycarboxylic acid, as described, e.g., in Elliott et al., WO 09/154737. In some embodiments, the boronic acid complexing agent is selected from the group consisting of glycolic acid, malic acid, hexahydromandelic acid, citric acid, 2-hydroxyisobutyric acid, 3-hydroxybutyric acid, mandelic acid, lactic acid, 2-hydroxy-3,3-dimethylbutyric acid, 2-hydroxy-3-methylbutyric acid, 2-hydroxyisocaproic acid, beta-hydroxyisovaleric acid, salicylic acid, tartaric acid, benzilic acid, glucoheptonic acid, maltonic acid, lactobionic acid, galactaric acid, embonic acid, 1-hydroxy-2-naphthoic acid, and 3-hydroxy-2-naphthoic acid. In certain embodiments, the boronic acid complexing agent is citric acid.

In certain embodiments, wherein the alpha-hydroxy carboxylic acid or beta-hydroxy carboxylic acid is citric acid, the compound of formula (***I***) is characterized by formula (***III-A***) or (***III-B***): 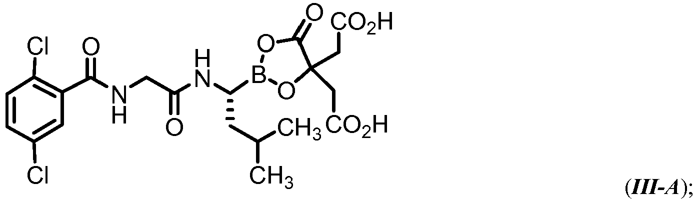 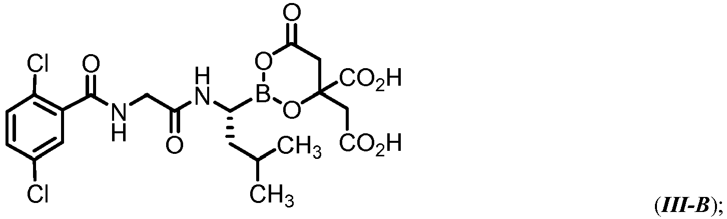 or a mixture thereof or a pharmaceutical composition thereof.

In certain embodiments, wherein the alpha-hydroxy carboxylic acid or beta-hydroxy carboxylic acid is citric acid, the compound of formula (***I***) is characterized by formula (***III-A***): 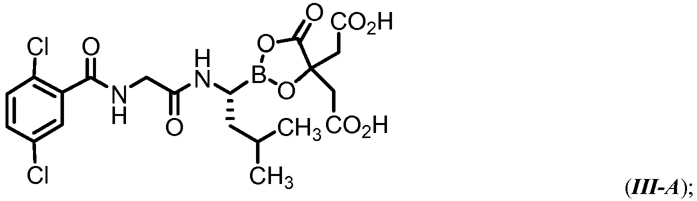 or a pharmaceutical composition thereof.

The compound of formula (***III-A***)*,* 2,2'-{2-[(1*R*)-1-({[(2,5-dichlorobenzoyl)amino]acetyl}amino)-3-methylbutyl]-5-oxo-1,3,2-dioxaborolane-4,4-diyl}diacetic acid (MLN9708) is disclosed in Elliott et al., WO 09/154737

In some embodiments, as set out in the claims the invention provides a method of determining whether to treat a patient with cancer with a therapeutically effective amount of a proteasome inhibitor or a pharmaceutically acceptable salt or a pharmaceutical composition thereof, based on identifying the patient with cancer as being likely to respond based upon an elevated level of NFκB p65 in the patient's tumor sample.

The cancers that can be treated with the present invention are colorectal cancer; squamous lung cancer, ovarian cancer, including, e.g., progressive epithelial or primary peritoneal cancer; gastric cancer; nasopharyngeal cancer and melanoma.

In some embodiments, the invention relates to a patient having or at risk of developing or experiencing a recurrence in a cancer selected from the group consisting of ovarian cancer, gastric cancer, nasopharyngeal cancer, squamous lung cancer, melanoma, and colorectal cancer.

In some embodiments, the compound or composition of the invention is used to treat a patient having or at risk of developing or experiencing a recurrence in a cancer selected from the group consisting of ovarian cancer, gastric cancer, nasopharyngeal cancer, lung cancer, melanoma and colorectal cancer. In an embodiment, the compound or composition of the invention is used to treat a patient having or at risk of developing or experiencing a recurrence in ovarian cancer. In an embodiment, the compound or composition of the invention is used to treat a patient having or at risk of developing or experiencing a recurrence in nasopharyngeal cancer. In an embodiment, the compound or composition of the invention is used to treat a patient having or at risk of developing or experiencing a recurrence in melanoma. In some embodiments, the lung cancer is adenocarcinoma or squamous cell carcinoma.

Splice variation of the NFκB p65 gene (ID 5970) results in isoforms of the NFκB p65 protein. Multiple alignment of four NFκB p65 isoforms is shown in Figure 2. Portions of NFκB p65 which are common to all isoforms are apparent upon review of Figure 2. In some embodiments, measurement of elevated NFκB p65, such as by the NFκB p65 IHC assay, comprises the use of an antibody which recognizes all isoforms of NFκB p65. For example, the detection antibody can be prepared to bind to a portion of NFκB p65 which is common to all isoforms. For example, the antibody can bind to the carboxy terminal (C-terminal) portion of NFκB p65 or the amino terminal (N-terminal) portion of NFκB p65. As used herein, the C-terminal portion of NFκB p65 consists of about 42 amino acid residues at the NFκB p65 C-terminus. Accordingly, an antibody which detects all isoforms of NFκB p65 can be generated using the sequence of the C-terminal portion, for example, about 10 to about 40 amino acid residues, about 30 to about 40 amino acid residues, at least 15 amino acid residues, at least 20 amino acid residues or at least 25 amino acid residues from the C-terminal portion of NFκB p65. As used herein, the N-terminal portion of NFκB p65 consists of about 150 amino acid residues at the NFκB p65 N-terminus. Accordingly, an antibody which detects all isoforms of NFκB p65 can be generated using the sequence of the N-terminal portion, for example, about 10 to about 150 amino acid residues, about 20 to about 100 amino acid residues, about at least 15 amino acid residues, at least 20 amino acid residues or at least 25 amino acid residues from the N-terminal portion of NFκB p65. Isoform 1 is the longest isoform of NFκB p65 and comprises segments present in all isoforms. Immunization with an isolated NFκB p65 isoform 1 can elicit antibodies which bind all isoforms.

Conversely, comparison of isoform sequences, such as in multiple alignment or pairwise alignment, can indicate regions of NFκB p65 which are not present in other isoforms. Accordingly, an antibody for an assay to measure only a single isoform can be generated to bind to a region, such as a splice junction, which is only in one isoform. Similarly, an antibody can be generated to bind to a region which is in some isoforms, but not others, e.g., a portion of isoform 1, SEQ ID NO:1 which is not present in SEQ ID NO:2, SEQ ID NO:3 or SEQ ID NO:4, a portion of SEQ ID NOs: 1 and 2 which is not present in SEQ ID NOs:3 or 4, or a portion of SEQ ID NOs: 1, 2 and 3 which is not present in SEQ ID NO:4.

In some embodiments, an assay to measure NFκB p65, such as the NFκB p65 IHC assay comprises the use of an antibody which binds to a protein with the sequence of SEQ ID NO:1. In some embodiments, the NFκB p65 assay comprises the use of an antibody which binds to a protein with the sequence of SEQ ID NO:2. In some embodiments, the NFκB p65 assay comprises the use of an antibody which binds to a protein with the sequence of SEQ ID NO:3. In some embodiments, the NFκB p65 assay comprises the use of an antibody which binds to a protein with the sequence of SEQ ID NO:4.

In some embodiments, an assay to measure NFκB p65, such as the NFκB p65 IHC assay measures the amount of SEQ ID NO:1. In some embodiments, the NFκB p65 assay measures the amount of SEQ ID NO:2. In some embodiments, the NFκB p65 assay measures the amount of SEQ ID NO:3. In some embodiments, the NFκB p65 assay measures the amount of SEQ ID NO:4. In some embodiments, the NFκB p65 assay measures the amount of all isoforms of the 65 kDa subunit of NF-κB. In an embodiment, an NFκB p65 assay measures the total amount of proteins with the sequences of SEQ ID NOs:1, 2, 3 and 4.

Due to variations among human populations, for detecting total NFκB p65, it might be advantageous to use an assay which is not sensitive to variation in NFκB p65 sequence, structure or post-translational modification. Accordingly, an assay to measure NFκB p65, such as the NFκB p65 IHC assay comprises the use of polyspecific antibody detection is disclosed. In some disclosures, the NFκB p65 assay comprises the use of a polyclonal antibody. The polyclonal antibody can be serum or minimally purified, e.g., from serum, milk or yolk, after immunization of an animal with an entire NFκB p65 protein or a portion thereof, the minimal purification to exclude non-immunoglobulin, e.g., serum, milk or yolk, proteins and/or antibodies which bind to proteins other than NFκB p65. In some disclosures, the NFκB p65 assay comprises the use of more than one, such as a pair or a population of antibodies generated against more than one specific portion of NFκB p65, to allow binding to more than one specific portion of NFκB p65.

Another advantage to using an assay, to measure NFκB p65, such as NFκB p65 IHC assay, which is not sensitive to variation is due to possible variation in sample preparation methods and sample quality. Since the method contemplates the use of archival samples, some degradation may occur prior to the assay. A balance can be struck between detecting all types or structures of NFκB p65 and keeping a high signal relative to background staining by several art-known immunochemistry, such as IHC, techniques. For example, the practitioner optionally may undertake pre-assay choices and/or IHC assay choices. Examples of pre-assay choices include choice of antibody, the step of eliminating cross-reaction, e.g., by preadsorbtion of the antibody preparation against a protein whose detection is not desired, choice of label or development reagent, and the method of fixation of the biological sample. Examples of IHC assay choices include method or choice of reagent for blocking non-specific binding, and the method or choice of reagent for washing tissue sections. Optimization of conditions for IHC assays is well known in the art, with techniques guides available, for example Buchwalow and Böcker, Immunohistochemistry Basics and Methods (2010, Springer-Verlag, Berlin, Germany).

Upon activation of the cytoplasmic (or cytosolic) NFκB complex comprising p65 and p50, the p65 transfers with p50 to the nucleus. In some embodiments, an assay to measure NFκB p65, such as the NFκB p65 assay measures the amount of NFκB p65 in the nucleus. In some embodiments, the NFκB p65 assay measures the amount of NFκB p65 in the cytoplasm. In some embodiments, the NFκB p65 assay measures the total (cytoplasmic + nuclear) amount of NFκB p65 in the cell, tumor section or biopsy sample.

The tumor sample is characterized by having a H-score as measured by a NFκB p65 IHC assay of between 250 and 300. The tumor sample may be characterized by having a H-score as measured by a NFκB p65 IHC assay of between 260 and 300. The tumor sample may be characterized by having a H-score as measured by a NFκB p65 IHC assay of between 270 and 300. The tumor sample may be characterized by having a H-score as measured by a NFκB p65 IHC assay of between 280 and 300. The tumor sample may be characterized by having a H-score as measured by a NFκB p65 IHC assay of between 290 and 300.

In some embodiments, the tumor sample is an archival tumor biopsy sample obtained either at or post-diagnosis. In some embodiments, the tumor sample is a fresh tumor biopsy. In some embodiments, the tumor sample is a fresh tumor biopsy obtained within 14 days of beginning treatment. In some embodiments, the tumor sample is a tumor biopsy obtained after 2 to 10 cycles, after about 4 cycles, after about 6 cycles, or after about 9 cycles of treatment. In some embodiments, the tumor sample is a tumor biopsy obtained after 2 to 10 cycles, after about 4 cycles, after about 6 cycles, or after about 9 cycles of treatment with the compound of formula (***III-A***) or a pharmaceutical composition thereof.

Tissue samples to assay for elevated NFκB p65, such as in a NFκB p65 IHC assay, can be obtained from a patient who is a candidate for treatment as described herein or from a commercial source. Commercial sources of tissue samples, such as tumor samples or control samples, include PhenoPath Laboratories, PLLC, Seattle, WA; Proteogenix SAS, Obershausbergen, France; and U.S. Biomax, Inc., Rockville, MD.

A sample for use in an assay to measure NFκB p65, such as the NFκB p65 IHC assay can be a biological sample comprising cells obtained from a patient afflicted with cancer. A sample can contain tumor cells or normal cells from the tissue or organ of cancer origin or a mixture of tumor cells and normal cells. A sample can be from a vicinity of a tumor tissue, such as a lymph node or from a distant tissue, such as liver or bone, to which the cancer may spread by metastasis. In some embodiments, the cancer sample is selected from the group consisting carcinoma, teratoma and sarcoma. A carcinoma tumor sample can be an adenocarcinoma or a squamous cell carcinoma.

In some embodiments, for patients with ovarian cancer the tumor sample comprises cells or fluids obtained from the patient. The cells may be found in an ovarian tissue sample collected, for example, by an ovarian tissue biopsy or histology section. In some embodiments, the patient sample is an ovary-associated body fluid. Such fluids include, for example, blood fluids, lymph, ascites fluids, gynecological fluids, cystic fluids, urine, and fluids collected by peritoneal rinsing. In some embodiments, the sample comprises cells obtained from the patient. In this embodiment, the cells may be found in a fluid selected from the group consisting of a fluid collected by peritoneal rinsing, a fluid collected by uterine rinsing, a uterine fluid, a uterine exudate, a pleural fluid, and ovarian exudates.

In some embodiments, for patients with lung cancer, the tumor sample is cells or fluids obtained from the patient. For example, the tumor sample may be obtained from sputum, tissue biopsy from lung or lymph node, or pleural effusions.

In some embodiments, for patients with colorectal cancer, the tumor sample comprises cells obtained from the patient. The cells may be found in a colon smear or biopsy of a polyp collected, for example, by colonoscopy. In some embodiments, the tumor sample is a body fluid. Such fluids include, for example, blood fluids, stool, colon lavage fluids and lymph fluids.

In some embodiments, the proteasome inhibitor or a pharmaceutically acceptable salt or a pharmaceutical composition thereof is administered orally. In some embodiments, the proteasome inhibitor or a pharmaceutically acceptable salt or a pharmaceutical composition is administered intraveneously. In some embodiments the proteasome inhibitor or a pharmaceutically acceptable salt or a pharmaceutical composition thereof is administered subcutaneously. In some embodiments, the compound of formula (***III-A***) or a pharmaceutical composition thereof is administered orally. In certain such embodiments, the compound of formula (***III-A***) or a pharmaceutical composition thereof is administered in a capsule. In some embodiments, the compounds of formula (***III-A***)*,* or a pharmaceutically acceptable salt or a pharmaceutical composition or a boronic acid anhydride thereof is administered intravenously. In certain such embodiments, the compound of formula (***III-A***) or a pharmaceutical composition thereof is administered intravenously.

In some embodiments, the compound of formula (***III-A***)*,* or a pharmaceutically acceptable salt or a pharmaceutical composition or a boronic acid anhydride thereof is administered on a weekly schedule. In some embodiments, the compound of formula (***III-A***) or a pharmaceutical composition thereof is administered on a weekly schedule. In some embodiments, the compound of formula (***III-**A*), or a pharmaceutically acceptable salt or a pharmaceutical composition or a boronic acid anhydride thereof is administered on days 1, 8, and 15 of a 28-day cycle. In some embodiments, the compound of formula (***III-A***) or a pharmaceutical composition thereof is administered on days 1, 8, and 15 of a 28-day cycle.

In some embodiments, the compound of formula (***III-A***)*,* or a pharmaceutically acceptable salt or a pharmaceutical composition or a boronic acid anhydride thereof is administered on a twice-weekly schedule. In some embodiments, the compound of formula (***III-A***) or a pharmaceutical composition thereof is administered on a twice-weekly schedule. In some embodiments, the compound of formula (***III-A***), or a pharmaceutically acceptable salt or a pharmaceutical composition or a boronic acid anhydride thereof is administered on days 1, 4, 8, and 11 of a 21-day cycle. In some embodiments, the compound of formula (***III-A***) or a pharmaceutical composition thereof is administered on days 1, 4, 8, and 11 of a 21-day cycle.

In some embodiments, the amount of the compound of formula (***III-A***) is about 0.2 mg to about 7.5 mg per dose based on the amount of the compound of formula (***II***). In some embodiments, the amount of the compound of formula (***III-A***) is about 1.6 mg to about 5.5 mg per dose based on the amount of the compound of formula (***II***). In some embodiments, the amount of the compound of formula (***III-A***) is about 2.3 mg to about 5.5 mg per dose based on the amount of the compound of formula (***II***).

In some embodiments, the proteasome inhibitor or a pharmaceutically acceptable salt or a pharmaceutical composition thereof is administered in conjunction with another therapeutic modality. In some embodiments, the compound of formula (***III-A***), or a pharmaceutically acceptable salt or a pharmaceutical composition or a boronic acid anhydride thereof is administered in conjunction with another therapeutic modality. In some embodiments, the compound of formula (***III-A***) or a pharmaceutical composition thereof is administered in conjunction with another therapeutic modality. In some embodiments, the therapeutic modality is one that is normally administered to patients with ovarian cancer, gastric cancer, nasopharyngeal cancer, squamous lung cancer, melanoma, or colorectal cancer. In some embodiments, the other therapeutic modality is radiotherapy. In some embodiments, the other therapeutic modality is another therapeutic agent. In some embodiments, the other therapeutic modality is radiotherapy and one or more therapeutic agents. The other therapeutic agent may be administered in the same dosage form or as a separate dosage form. When administered as a separate dosage form, the other therapeutic agent may be administered prior to, at the same time as, or following administration of the proteasome inhibitor or a pharmaceutically acceptable salt or a pharmaceutical composition thereof.

Non-limiting examples of therapeutic agents include DNA damaging chemotherapeutic agents include topoisomerase I inhibitors (e.g., irinotecan, topotecan, camptothecin and analogs or metabolites thereof, and doxorubicin); topoisomerase II inhibitors (e.g., etoposide, teniposide, epirubicin, and daunorubicin); alkylating agents (e.g., melphalan, chlorambucil, busulfan, thiotepa, ifosfamide, carmustine, lomustine, semustine, streptozocin, decarbazine, methotrexate, mitomycin C, and cyclophosphamide); DNA intercalators (e.g., cisplatin, oxaliplatin, and carboplatin); DNA intercalators and free radical generators such as bleomycin; and nucleoside mimetics (e.g., 5-fluorouracil, capecitibine, gemcitabine, fludarabine, cytarabine, mercaptopurine, thioguanine, pentostatin, and hydroxyurea).

Other non-limiting examples of therapeutic agents include chemotherapeutic agents that disrupt cell replication include: paclitaxel, docetaxel, and related analogs; vincristine, vinblastin, and related analogs; thalidomide, lenalidomide, and related analogs (e.g., CC-5013 and CC-4047); protein tyrosine kinase inhibitors (e.g., imatinib mesylate, erlotonib, sorafenib, crizitonib, vemurafenib and gefitinib); proteasome inhibitors (e.g., bortezomib); NF-κB inhibitors, including inhibitors of IκB kinase; antibodies which bind to proteins overexpressed in cancers and thereby downregulate cell replication (e.g., trastuzumab, rituximab, cetuximab, panitumumab, ipilimumab, and bevacizumab); and other inhibitors of proteins or enzymes known to be upregulated, over-expressed or activated in cancers, the inhibition of which downregulates cell replication.

Radiotherapy may be used as another therapeutic modality prior to, at the same time as, or following administration of the proteasome inhibitor or a pharmaceutically acceptable salt or a pharmaceutical composition thereof. In some embodiments, the radiotherapy is external beam radiotherapy. External beam radiotherapy is given as a series of treatments known as fractions. In some such embodiments, the external beam radiotherapy is conformal radiotherapy. In some embodiments, the radiotherapy is internal radiotherapy. Internal radiotherapy uses a radioactive substance sealed in needles, seeds, wires, or catheters that are placed directly into or near the cancer.

### Formulation and Administration

If a pharmaceutically acceptable salt of a proteasome inhibitor is utilized in these compositions, the salt preferably is derived from an inorganic or organic acid or base. For reviews of suitable salts, see, e.g., Berge et al, J. Pharm. Sci. 66:1-19 (1977) and Remington: The Science and Practice of Pharmacy, 20th Ed., ed. A. Gennaro, Lippincott Williams & Wilkins, 2000.

Nonlimiting examples of suitable acid addition salts include the following: acetate, adipate, alginate, aspartate, benzoate, benzene sulfonate, bisulfate, butyrate, citrate, camphorate, camphor sulfonate, cyclopentanepropionate, digluconate, dodecylsulfate, ethanesulfonate, fumarate, lucoheptanoate, glycerophosphate, hemisulfate, heptanoate, hexanoate, hydrochloride, hydrobromide, hydroiodide, 2-hydroxyethanesulfonate, lactate, maleate, methanesulfonate, 2-naphthalenesulfonate, nicotinate, oxalate, pamoate, pectinate, persulfate, 3-phenyl-propionate, picrate, pivalate, propionate, succinate, tartrate, thiocyanate, tosylate and undecanoate.

Suitable base addition salts include, without limitation, ammonium salts, alkali metal salts, such as lithium, sodium and potassium salts; alkaline earth metal salts, such as calcium and magnesium salts; other multivalent metal salts, such as zinc salts; salts with organic bases, such as dicyclohexylamine, *N*-methyl-D-glucamine, *t*-butylamine, ethylene diamine, ethanolamine, and choline; and salts with amino acids such as arginine, lysine, and so forth. In some embodiments, the pharmaceutically acceptable salt is a base addition salt of a boronic acid compound of formula (***I***), wherein Z¹ and Z² are both hydroxy.

The term "pharmaceutically acceptable carrier" is used herein to refer to a material that is compatible with a recipient subject, preferably a mammal, more preferably a human, and is suitable for delivering an active agent to the target site without terminating the activity of the agent. The toxicity or adverse effects, if any, associated with the carrier preferably are commensurate with a reasonable risk/benefit ratio for the intended use of the active agent.

The terms "carrier", "adjuvant", or "vehicle" are used interchangeably herein, and include any and all solvents, diluents, and other liquid vehicles, dispersion or suspension aids, surface active agents, pH modifiers, isotonic agents, thickening or emulsifying agents, preservatives, solid binders, lubricants and the like, as suited to the particular dosage form desired. Remington: The Science and Practice of Pharmacy, 20th Ed., ed. A. Gennaro, Lippincott Williams & Wilkins, 2000 discloses various carriers used in formulating pharmaceutically acceptable compositions and known techniques for the preparation thereof. Except insofar as any conventional carrier medium is incompatible with the compounds of the invention, such as by producing any undesirable biological effect or otherwise interacting in a deleterious manner with any other component(s) of the pharmaceutically acceptable composition, its use is contemplated to be within the scope of this invention. Some examples of materials which can serve as pharmaceutically acceptable carriers include, but are not limited to, ion exchangers, alumina, aluminum stearate, lecithin, serum proteins, such as human serum albumin, buffer substances such as phosphates, carbonates, magnesium hydroxide and aluminum hydroxide, glycine, sorbic acid, or potassium sorbate, partial glyceride mixtures of saturated vegetable fatty acids, water, pyrogen-free water, salts or electrolytes such as protamine sulfate, disodium hydrogen phosphate, potassium hydrogen phosphate, sodium chloride, and zinc salts, colloidal silica, magnesium trisilicate, polyvinyl pyrrolidone, polyacrylates, waxes, polyethylene-polyoxypropylene-block polymers, wool fat, sugars such as lactose, glucose, sucrose, and mannitol, starches such as corn starch and potato starch, cellulose and its derivatives such as sodium carboxymethyl cellulose, ethyl cellulose and cellulose acetate, powdered tragacanth; malt, gelatin, talc, excipients such as cocoa butter and suppository waxes, oils such as peanut oil, cottonseed oil, safflower oil, sesame oil, olive oil, corn oil and soybean oil, glycols such as propylene glycol and polyethylene glycol, esters such as ethyl oleate and ethyl laurate, agar, alginic acid, isotonic saline, Ringer's solution, alcohols such as ethanol, isopropyl alcohol, hexadecyl alcohol, and glycerol, cyclodextrins such as hydroxypropyl β-cyclodextrin and sulfobutylether β-cyclodextrin, lubricants such as sodium lauryl sulfate and magnesium stearate, petroleum hydrocarbons such as mineral oil and petrolatum. Coloring agents, releasing agents, coating agents, sweetening, flavoring and perfuming agents, preservatives and antioxidants can also be present in the composition, according to the judgment of the formulator.

The pharmaceutical compositions utilized in the invention can be manufactured by methods well known in the art such as conventional granulating, mixing, dissolving, encapsulating, lyophilizing, or emulsifying processes, among others. Compositions may be produced in various forms, including granules, precipitates, or particulates, powders, including freeze dried, rotary dried or spray dried powders, amorphous powders, tablets, capsules, syrup, suppositories, injections, emulsions, elixirs, suspensions or solutions.

The pharmaceutical compositions utilized in the invention are formulated for pharmaceutical administration to a mammal, preferably a human being. Such pharmaceutical compositions of the present invention may be administered orally, parenterally, by inhalation spray, topically, rectally, nasally, buccally, vaginally or via an implanted reservoir. The term "parenteral" as used herein includes subcutaneous, intravenous, intramuscular, intra-articular, intra-synovial, intrasternal, intrathecal, intrahepatic, intralesional and intracranial injection or infusion techniques. Preferably, the compositions are administered orally, intravenously, or subcutaneously. The formulations of the invention may be designed to be short-acting, fast-releasing, or long-acting. Still further, compounds can be administered in a local rather than systemic means, such as administration (e.g., by injection) at a tumor site.

Liquid dosage forms for oral administration include, but are not limited to, pharmaceutically acceptable emulsions, microemulsions, solutions, suspensions, syrups and elixirs. In addition to the active compounds, the liquid dosage forms may contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, cyclodextrins, dimethylformamide, oils (in particular, cottonseed, groundnut, corn, germ, olive, castor, and sesame oils), glycerol, tetrahydrofurfuryl alcohol, polyethylene glycols and fatty acid esters of sorbitan, and mixtures thereof. Besides inert diluents, the oral compositions can also include adjuvants such as wetting agents, emulsifying and suspending agents, sweetening, flavoring, and perfuming agents.

Injectable preparations, for example, sterile injectable aqueous or oleaginous suspensions may be formulated according to the known art using suitable dispersing or wetting agents and suspending agents. The sterile injectable preparation may also be a sterile injectable solution, suspension or emulsion in a nontoxic parenterally acceptable diluent or solvent, for example, as a solution in 1,3-butanediol. Among the acceptable vehicles and solvents that may be employed are water, Ringer's solution, U.S.P. and isotonic sodium chloride solution. In addition, sterile, fixed oils are conventionally employed as a solvent or suspending medium. For this purpose any bland fixed oil can be employed including synthetic mono- or diglycerides. In addition, fatty acids such as oleic acid are used in the preparation of injectables. The injectable formulations can be sterilized, for example, by filtration through a bacterial-retaining filter, or by incorporating sterilizing agents in the form of sterile solid compositions which can be dissolved or dispersed in sterile water or other sterile injectable medium prior to use. Compositions formulated for parenteral administration may be injected by bolus injection or by timed push, or may be administered by continuous infusion.

Solid dosage forms for oral administration include capsules, tablets, pills, powders, and granules. In such solid dosage forms, the active compound is mixed with at least one inert, pharmaceutically acceptable excipient or carrier such as sodium citrate or dicalcium phosphate and/or a) fillers or extenders such as starches, lactose, sucrose, glucose, mannitol, and silicic acid, b) binders such as, for example, carboxymethylcellulose, alginates, gelatin, polyvinylpyrrolidinone, sucrose, and acacia, c) humectants such as glycerol, d) disintegrating agents such as agar--agar, calcium carbonate, potato or tapioca starch, alginic acid, certain silicates, and sodium carbonate, e) solution retarding agents such as paraffin, f) absorption accelerators such as quaternary ammonium compounds, g) wetting agents such as, for example, cetyl alcohol and glycerol monostearate, h) absorbents such as kaolin and bentonite clay, and i) lubricants such as talc, calcium stearate, magnesium stearate, solid polyethylene glycols, sodium lauryl sulfate, and mixtures thereof. In the case of capsules, tablets and pills, the dosage form may also comprise buffering agents such as phosphates or carbonates.

Solid compositions of a similar type may also be employed as fillers in soft and hard-filled gelatin capsules using such excipients as lactose or milk sugar as well as high molecular weight polyethylene glycols and the like. The solid dosage forms of tablets, dragees, capsules, pills, and granules can be prepared with coatings and shells such as enteric coatings and other coatings well known in the pharmaceutical formulating art. They may optionally contain opacifying agents and can also be of a composition that they release the active ingredient(s) only, or preferentially, in a certain part of the intestinal tract, optionally, in a delayed manner. Examples of embedding compositions that can be used include polymeric substances and waxes. Solid compositions of a similar type may also be employed as fillers in soft and hard-filled gelatin capsules using such excipients as lactose or milk sugar as well as high molecular weight polethylene glycols and the like.

In some embodiments, the compound of formula (***I***) is administered orally. In some embodiments, the compound of formula (***III-A***) or a pharamaceutical composition thereof is administered orally. In some such embodiments, a pharmaceutical composition of the compound of formula (***III-A***) is prepared in gelatin capsules as described in Elliott et al., WO 09/154737, herein incorporated by reference in its entirety. In some embodiments, the pharmaceutical composition comprises the compound of formula (***III-A***) or a crystalline form thereof, a filler, optionally a lubricant, optionally a flow-aid and optionally a buffer. In some embodiments, the pharmaceutical composition comprises the compound of formula (***III-A***) or a crystalline form thereof, a filler, a lubricant, and a flow-aid. In some embodiments, the pharmaceutical composition comprises about 0.2% to about 12% of the compound of formula (***III-A***)*,* or a crystalline form thereof, about 76.5% to about 99.8% of a filler, optionally up to about 1.5% of a lubricant, and optionally up to about 5% of a flow-aid. The oral pharmaceutical compositions can be prepared by methods described in Elliott et al., WO 09/154737, herein incorporated by reference in its entirety.

The active compounds can also be in micro-encapsulated form with one or more excipients as noted above. The solid dosage forms of tablets, dragees, capsules, pills, and granules can be prepared with coatings and shells such as enteric coatings, release controlling coatings and other coatings well known in the pharmaceutical formulating art. In such solid dosage forms the active compound may be admixed with at least one inert diluent such as sucrose, lactose or starch. Such dosage forms may also comprise, as is normal practice, additional substances other than inert diluents, e.g., tableting lubricants and other tableting aids such a magnesium stearate and microcrystalline cellulose. In the case of capsules, tablets and pills, the dosage forms may also comprise buffering agents. They may optionally contain opacifying agents and can also be of a composition that they release the active ingredient(s) only, or preferentially, in a certain part of the intestinal tract, optionally, in a delayed manner. Examples of embedding compositions that can be used include polymeric substances and waxes.

Dosage forms for topical or transdermal administration of a compound of this invention include ointments, pastes, creams, lotions, gels, powders, solutions, sprays, inhalants or patches. The active component is admixed under sterile conditions with a pharmaceutically acceptable carrier and any needed preservatives or buffers as may be required. Ophthalmic formulation, ear drops, and eye drops are also contemplated as being within the scope of this invention. Additionally, the present invention contemplates the use of transdermal patches, which have the added advantage of providing controlled delivery of a compound to the body. Such dosage forms can be made by dissolving or dispensing the compound in the proper medium. Absorption enhancers can also be used to increase the flux of the compound across the skin. The rate can be controlled by either providing a rate controlling membrane or by dispersing the compound in a polymer matrix or gel.

In some embodiments, the proteasome inhibitor is administered intravenously. In some embodiments, the compound of formula (***III-A***) is administered intravenously. In some such embodiments, the compound wherein Z¹ and Z² together form a moiety derived from a boronic acid complexing agent can be prepared in the form of a lyophilized powder, as described in Plamondon et al., WO 02/059131 or Elliott et al., WO 09/154737. In some embodiments, the lyophilized powder also comprises free boronic acid complexing agent. Preferably, the free boronic acid complexing agent and the compound of formula (***III-A***) are present in the mixture in a molar ratio ranging from about 0.5:1 to about 100:1, more preferably from about 5:1 to about 100:1. In various embodiments, the lyophilized powder comprises free boronic acid complexing agent and the corresponding boronate ester in a molar ratio ranging from about 10:1 to about 100:1, from about 20:1 to about 100:1, or from about 40:1 to about 100:1.

In some embodiments, the lyophilized powder comprises boronic acid complexing agent and a compound of formula (***I***), substantially free of other components. However, the composition can further comprise one or more other pharmaceutically acceptable excipients, carriers, diluents, fillers, salts, buffers, bulking agents, stabilizers, solubilizers, and other materials well known in the art. The preparation of pharmaceutically acceptable formulations containing these materials is described in, *e.g.,* Remington: The Science and Practice of Pharmacy, 20th Ed., ed. A. Gennaro, Lippincott Williams & Wilkins, 2000, or latest edition. In some embodiments, the pharmaceutical composition comprises a compound of formula (***III-A***)*,* a bulking agent, and a buffer.

The lyophilized powder comprising the compound of formula (***III-A***) can be prepared according to the methods described in Plamondon et al., WO 02/059131 or Elliott et al., WO 09/154737. In some embodiments, the method for preparing the lyophilized powder comprises: (a) preparing an aqueous mixture comprising a boronic acid compound of formula (***I***), wherein Z¹ and Z² are each hydroxy, and a boronic acid complexing agent; and (b) lyophilizing the mixture. In some embodiments, the method for preparing the lyophilized powder comprises: (a) preparing an aqueous mixture comprising the compound of formula (***III-A***)*,* a bulking agent, and a buffer; and (b) lyophilizing the mixture.

The lyophilized powder preferably is reconstituted by adding an aqueous solvent suitable for pharmaceutical administrations. Examples of suitable reconstitution solvents include, without limitation, water, saline, and phosphate buffered saline (PBS). Preferably, the lyophilized powder is reconstituted with normal (0.9%) saline. Upon reconstitution, an equilibrium is established between a boronate ester compound and the corresponding free boronic acid compound. In some embodiments, equilibrium is reached quickly, e.g., within 10-15 minutes, after the addition of aqueous medium. The relative concentrations of boronate ester and boronic acid present at equilibrium is dependent upon parameters such as, e.g., the pH of the solution, temperature, the nature of the boronic acid complexing agent, and the ratio of boronic acid complexing agent to boronate ester compound present in the lyophilized powder.

The pharmaceutical compositions utilized in the present invention preferably are formulated for administration to a patient having, or at risk of developing or experiencing a recurrence of, cancer. Preferred pharmaceutical compositions utilized in the present invention are those formulated for oral, intravenous, or subcutaneous administration. Any of the above dosage forms containing a therapeutically effective amount of a proteasome inhibitor are well within the bounds of routine experimentation and within the scope of the present invention. In some embodiments, the pharmaceutical composition utilized in the present invention may further comprise another therapeutic agent.

The amount of additional therapeutic agent present in a composition of this invention typically will be no more than the amount that would normally be administered in a composition comprising that therapeutic agent as the only active agent. Preferably, the amount of additional therapeutic agent will range from about 50% to about 100% of the amount normally present in a composition comprising that agent as the only therapeutically active agent.

In order that this invention be more fully understood, the following preparative and testing examples are set forth. These examples illustrate how to make or test specific compounds, and are not to be construed as limiting the scope of the invention in any way.

### EXAMPLES

### Example 1: Preparation of compounds and pharmaceutical compositions

The compound of formula (***II***), [(1R)-1-({[(2,5-dichlorobenzoyl)amino]acetyl}amino)-3-methylbutyl]boronic acid, is prepared by methods disclosed in Olhava and Danca, U.S. Patent No. 7,442,830, herein incorporated by reference in its entirety. The compound of formula (***III-A***)*,* 2,2'-{2-[(1*R*)-1-({[(2,5-dichlorobenzoyl)amino]acetyl}amino)-3-methylbutyl]-5-oxo-1,3,2-dioxaborolane-4,4-diyl}diacetic acid, is prepared by methods disclosed in Elliott et al., WO 09/154737, herein incorporated by reference in its entirety. An oral capsule formulation of the compound of formula (***III-A***) is prepared by methods disclosed in Elliott et al., WO 09/154737, herein incorporated by reference in its entirety. An IV formulation of the compound of formula (***III-A***) is prepared by methods disclosed in Elliott et al., WO 09/154737, herein incorporated by reference in its entirety. A lyophilized formulation of the compound of formula (***III-A***) suitable for reconstitution into an IV formulation is prepared by methods disclosed in Elliott et al., WO 09/154737, herein incorporated by reference in its entirety.

### Example 2: Measurement of cytosolic NFκB p65 IHC assay H-score - Assay 1

A previously prepared formalin fixed paraffin embedded (FFPE) immunohistochemistry (IHC) slide is deparaffinized using xylene and graded alcohols on a Sakura DRS slide stainer, and then incubated for 5 minutes in 3% H₂O₂. The slide is treated with steam for 30 minutes in a citrate buffer at pH6. The slide is then treated with a 1:50 solution of a NPκB p65 antigen rabbit monoclonal antibody [Cell Signaling Technologies, clone (C22B4) #4764] overnight in a humid chamber. After overnight incubation the slide is washed and placed on a Dako Autostainer and developed with a secondary antibody (Ultravision, Thermo Fisher) and DAB+ substrate (Dako). After development, the slide is washed in de-ionized water, counterstained using Meyer's hematoxylin, dehydrated and coverslipped.

Each slide is evaluated by the pathologist for nuclear and cytoplasmic signal including intensity of staining (0-3+) and percentage of positive cells in deciles to determine an H-score.

FIGURE 1 displays the results of the above assay on the following:
(1) Tissue Microarray (obtained from US Biomax, Inc.) of primary nasopharyngeal cancer (n = 35)
(2) Tissue Microarray of metastatic nasopharyngeal cancer (obtained from US Biomax, Inc.) (n = 15)
(3) Tissue Microarray of other histologies of head and neck cancers (obtained from US Biomax, Inc.) (n = 68)
(4) Archival primary tumor biopsy samples of patients with head and neck squamous cell cancers (n = 14). All samples were obtained under the appropriate informed consent. The patients were administered the compound of formula (***III-A***) on a twice-weekly schedule (days 1, 4, 8 and 11 of a 21-day cycle) as an IV injection.

Of the primary tumor samples in (4), there were three patients with nasopharyngeal cancer. Patient 1 with metastatic nasopharyngeal cancer of a squamous cell type had a durable partial response; Patient 2's disease progressed within 4 cycles; and Patient 3 had progressive disease. These three patients showed the following NPκB p65 IHC assay H-scores (Table 1).

**Table 1: NFκB p65 IHC assay H-score values for patients with nasopharyngeal cancer**

| **Patient** | **Responder/Non-responder** | **NFκB p65 IHC assay H-score** |
|---|---|---|
| Patient 1 | Responder | 300 |
| Patient 2 | Non-responder | 200 |
| Patient 3 | Non-responder | 160 |

### Example 3: Measurement of cytosolic NPκB p65 IHC assay H-score - Assay 2

A previously prepared FFPE IHC slide is deparaffinized using xylene and graded alcohols on a Sakura DRS slide stainer, and then incubated for 5 minutes in 3% H₂O₂. The slide is treated with steam for 30 minutes in a citrate buffer at pH6. The slide is then treated with a 1:200 solution of a NPκB p65 antigen goat polyclonal antibody [Santa Cruz, sc-372-G] for 1 hour on a Dako Autostainer. After 1 hour incubation the slide is developed with a secondary antibody (Bio-goat Elite (Vector) and DAB+ substrate (Dako). After development, the slide is washed in de-ionized water, counterstained using Meyer's hematoxylin, dehydrated and coverslipped.

Each slide is evaluated by the pathologist for nuclear and cytoplasmic signal including intensity of staining (0-3+) and percentage of positive cells in deciles to determine an H-score.

### Example 4: Measurement of cytosolic NFκB p65 IHC assay H-score - Assay 3

A previously prepared FFPE IHC slide is deparaffinized using xylene and graded alcohols on a Sakura DRS slide stainer, and then incubated for 5 minutes in 3% H₂O₂. The slide is treated in a microwave pressure cooker for 8 minutes in a citrate buffer at pH6. The slide is then treated with a 1:200 solution of a NFκB p65 antigen rabbit polyclonal antibody [Invitrogen, 51-0500] for 1 hour on a Dako Autostainer. After 1 hour incubation the slide is developed with a secondary antibody (Ultravision, Thermo Fisher) and DAB+ substrate (Dako). After development, the slide is washed in de-ionized water, counterstained using Meyer's hematoxylin, dehydrated and coverslipped.

Each slide is evaluated by the pathologist for nuclear and cytoplasmic signal including intensity of staining (0-3+) and percentage of positive cells in deciles to determine an H-score.

### Example 5: Measurement of cytosolic NFκB p65 IHC assay H-score in tissue samples

Tissue microarrays (obtained from US Biomax, Inc.) were tested using the NPκB p65 IHC assay procedure described in Example 2 above. Table 2 displays the percentage of samples that had a cytoplasmic H-score of greater than 200.

**Table 2. Prevalence of Tumors of Different Cancer Types with Elevated NPκB p65 on Tissue Microarray**

| **Cancer (number of samples)** | **Percent (%) of samples with a H-score of greater than 200** |
|---|---|
| Ovarian (n=186): | 28 |
| Melanoma (n=184): | 16.3 |
| C olorectal (n=188): | 18.6 |
| Lung Squamous. (n=71): | 19.7 |
| Nasopharyngeal (n=35): | 31.4 |
| Gastric (n=99): | 12.1 |
| Prostate (n=174) | 10.9 |
| Head & Neck (n=67) | 8.96 |
| Lung Adenocarcinoma (n=74) | 5.41 |
| Pancreatic (n=87) | 5.75 |
| Renal (n=134) | 0 |
| Sarcoma (n=139) | 9.35 |
| Breast (n=161) | 2 |
| SCLC (n=105) | 0.95 |

### Example 6. Comparison of Section Size for Measurement of NPκB p65

Whole tissue sections of various tumor types were obtained from commercial sources. NFκB p65 IHC assay was performed by the method described in Example 2. The prevalence of samples with an H score >200 is listed in Table 3 below.

**Table 3. Prevalence of Tumors with NPκB p65 IHC Assay H scores >200**

| **Cancer** | **H> 200 Sections (number of samples)** |
|---|---|
| Squamous lung | 24% (n=49) |
| Colon | 30% (n=47) |
| Melanoma | 36% (n=73) |
| Ovarian | 68% (n=50) |
| Gastric | 28% (n=18) |
| Nasopharyngeal | 31% (n=35) |
| Other Tumors: | |
| Breast | 16% (n=49) |
| HNSCC | 12% (n=42) |
| Lung adenocarcinoma | 5% (n=21) |

The results in Table 3 show that for most of the 9 tumor types tested the prevalence of marker positive on whole tumor sections is close to that seen for the small core sample used for a tissue microarray in Table 2.

### Example 7. Measurement of NFκB p65 on Whole tissue sections

When the NPκB p65 IHC Assay was performed by the method described in Example 2 on a larger group of whole sections of commercially available primary human tumors the results presented in Table 4 were obtained.

**Table 4. Prevalence of Whole Section Tumors with NPκB p65 IHC Assay H scores >200.**

| Tumor Type | p65 H score>200 (95% Confidence interval (CI)) (number of samples) |
|---|---|
| Gastric | 20% (95% CI: 5.7% - 35.4%) (n=35) |
| Squamous lung | 22.4% (95% CI: 10.2% - 32.7%) (n=49) |
| Colon | 25.4% (95% CI: 14.9% - 35.8%) (n=67) |
| Nasopharyngeal | 48.6% (95% CI: 37.1% - 60%) (n=70) |
| Melanoma | 36% (95% CI: 25% - 47.2%) (n=73) |
| Ovarian | 65.7% (95% CI: 54.3% - 77.1%) (n=70) |

As can be seen by the results in Table 4, ovarian and nasopharyngeal cancers are tumor indications where 50% or more tumor samples express high levels of cytosolic NPκB p65.

## Claims

1. A proteasome inhibitor, or a pharmaceutically acceptable salt or pharmaceutical composition thereof, for use in a method of treating cancer in a patient whose tumor sample is **characterized by** having a H-score of between 250 and 300, as measured by a nuclear factor kappa-B (NFκB p65) immunohistochemistry (IHC) assay, said method comprising administering a therapeutically effective amount of the proteasome inhibitor to said patient, wherein:
the proteasome inhibitor is **characterized by** the compound of formula (***III-A***): 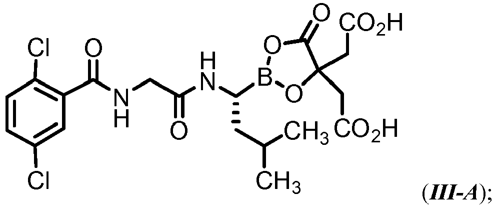 or a pharmaceutically acceptable salt or a pharmaceutical composition thereof;
wherein the cancer is selected from the group consisting of ovarian cancer, gastric cancer, nasopharyngeal cancer, squamous lung cancer, melanoma, and colorectal cancer.

2. A method for determining whether to treat a patient with cancer with a proteasome inhibitor or a pharmaceutically acceptable salt or pharmaceutical composition thereof; comprising:
a) measuring the level of NFκB p65 in a tumor sample from the patient as a H-score, wherein the H-score is determined by a NFκB p65 IHC assay; and
b) determining to treat the patient with a therapeutically effective amount of the proteasome inhibitor or a pharmaceutically acceptable salt or a pharmaceutical composition thereof, if the tumor sample is **characterized by** having a H-score of between 250 and 300, as measured by a NPκB p65 IHC assay, wherein:
the proteasome inhibitor is **characterized by** the compound of formula (***III-A***): 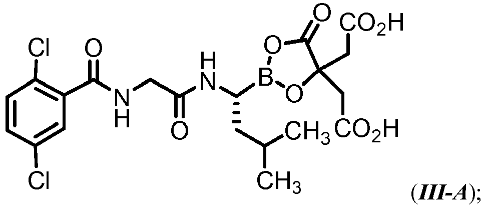 or a pharmaceutically acceptable salt or a pharmaceutical composition thereof;
wherein the cancer is selected from the group consisting of ovarian cancer, gastric cancer, nasopharyngeal cancer, squamous lung cancer, melanoma, and colorectal cancer.

3. The compound for use or the method according to any one of the preceding claims, wherein the NFκB p65 IHC assay comprises the use of an antibody which binds to a protein with the sequence of SEQ ID NO:1.

4. The compound for use according to claim 1 or the method according to claim 2, wherein the NFκB p65 IHC assay measures the total amount of proteins with the sequences of SEQ ID NOs:1, 2, 3 and 4.

5. The compound for use or the method according to any one of the preceding claims, wherein the NFκB p65 IHC assay measures the amount of the NFκB p65 present in the cytoplasm.

6. The compound for use or the method according to any one of the preceding claims, wherein the compound of formula (***III-A***) is administered orally or intravenously.

7. The compound for use or the method according to any one of the preceding claims, wherein the compound of formula (***III-A***) is administered on days 1, 8, and 15 of a 28-day cycle or on days 1, 4, 8 and 11 of a 21-day cycle.

8. The compound for use or the method according to any one of the preceding claims, wherein the compound of formula (***III-A***) is administered orally in one or more capsules.

9. The compound for use or the method according to any one of the preceding claims, wherein the amount of the compound of formula (***III-A***) is 2.3 mg to 5.5 mg per dose based on the amount of the compound of formula (***II***);

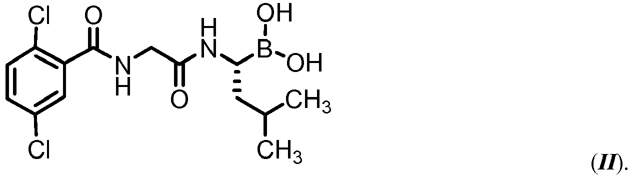

## Patentansprüche

1. Proteasomenhemmer oder pharmazeutisch unbedenkliches Salz oder pharmazeutische Zusammensetzung davon zur Verwendung bei einem Verfahren zur Behandlung von Krebs bei einem Patienten, dessen Tumorprobe **dadurch gekennzeichnet ist, dass** sie einen H-Score zwischen 250 und 300 aufweist, wie mit einem immunhistochemischen (IHC-) NFκB-p65(Nuclear Factor kappa-B)-Test gemessen, wobei das Verfahren Verabreichen einer therapeutisch wirksamen Menge des Proteasomenhemmers an den Patienten umfasst, wobei:
der Proteasomenhemmer durch die Verbindung der Formel (***III-A***): 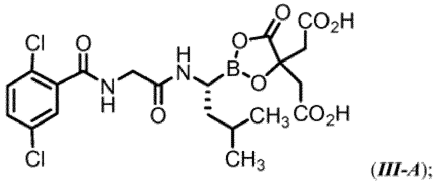 oder ein pharmazeutisch unbedenkliches Salz oder eine pharmazeutische Zusammensetzung davon gekennzeichnet ist;
wobei der Krebs ausgewählt ist aus der Gruppe bestehend aus Ovarialkarzinom, Magenkarzinom, Nasopharynxkarzinom, Plattenepithelkarzinom der Lunge, Melanom und Kolorektalkarzinom.

2. Verfahren zur Bestimmung davon, ob ein Patient mit Krebs mit einem Proteasomenhemmer oder einem pharmazeutisch unbedenklichen Salz bzw. einer pharmazeutischen Zusammensetzung davon behandelt wird; umfassend:
a) Messen des Spiegels von NFκB-p65 in einer Tumorprobe vom Patienten als H-Score, wobei der H-Score mit einem NFκB-p65-IHC-Test bestimmt wird; und
b) Bestimmen, dass der Patient mit einer therapeutisch wirksamen Menge des Proteasomen-ihemmers oder eines pharmazeutisch unbedenk-lichen Salzes oder einer pharmazeutischen Zusammen-isetzung davon behandelt wird, falls die Tumorprobe **dadurch gekennzeichnet ist, dass** sie einen H-Score zwischen 250 und 300 aufweist, wie mit einem NFκB-p65-IHC-Test gemessen, wobei:
der Proteasomenhemmer durch die Verbindung der Formel (***III-A***): 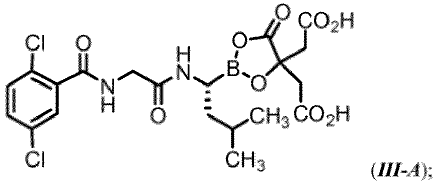 oder ein pharmazeutisch unbedenkliches Salz oder eine pharmazeutische Zusammensetzung davon gekennzeichnet ist;
wobei der Krebs ausgewählt ist aus der Gruppe bestehend aus Ovarialkarzinom, Magenkarzinom, Nasopharynxkarzinom, Plattenepithelkarzinom der Lunge, Melanom und Kolorektalkarzinom.

3. Verbindung zur Verwendung oder Verfahren nach einem der vorhergehenden Ansprüche, wobei der NFκB-p65-IHC-Test die Verwendung eines Antikörpers umfasst, der an ein Protein mit der Sequenz unter SEQ ID NO:1 bindet.

4. Verbindung zur Verwendung nach Anspruch 1 oder Verfahren nach Anspruch 2, wobei im NFκB-p65-IHC-Test die Gesamtmenge der Proteine mit den Sequenzen unter SEQ ID NO:1, 2, 3 und 4 gemessen wird.

5. Verbindung zur Verwendung oder Verfahren nach einem der vorhergehenden Ansprüche, wobei im NFκB-p65-IHC-Test die Menge des im Cytoplasma vorliegenden NFκB-p65 gemessen wird.

6. Verbindung zur Verwendung oder Verfahren nach einem der vorhergehenden Ansprüche, wobei die Verbindung der Formel (***III-A***) oral oder intravenös verabreicht wird.

7. Verbindung zur Verwendung oder Verfahren nach einem der vorhergehenden Ansprüche, wobei die Verbindung der Formel (***III-A***) an Tag 1, 8 und 15 eines 28-Tage-Zyklus oder an Tag 1, 4, 8 und 11 eines 21-Tage-Zyklus verabreicht wird.

8. Verbindung zur Verwendung oder Verfahren nach einem der vorhergehenden Ansprüche, wobei die Verbindung der Formel (***III-A***) oral in einer oder mehreren Kapseln verabreicht wird.

9. Verbindung zur Verwendung oder Verfahren nach einem der vorhergehenden Ansprüche, wobei die Menge der Verbindung der Formel (***III-A***) 2,3 mg bis 5,5 mg pro Dosis bezogen auf die Menge der Verbindung der Formel (***II***) beträgt;

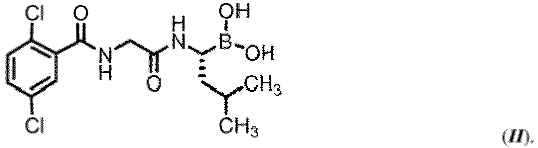

## Revendications

1. Inhibiteur de protéasome, ou sel pharmaceutiquement acceptable ou composition pharmaceutique de celui-ci, pour utilisation dans une méthode de traitement du cancer chez un patient dont l'échantillon tumoral est **caractérisé en ce qu'**il présente un score H compris entre 250 et 300, comme mesuré par un dosage d'immunohistochimie (IHC) du facteur nucléaire kappa-B (NFκB p65), ladite méthode comprenant l'administration d'une quantité thérapeutiquement active de l'inhibiteur de protéasome audit patient, où :
l'inhibiteur de protéasome est **caractérisé par** le composé de formule ***(III-A)*** : 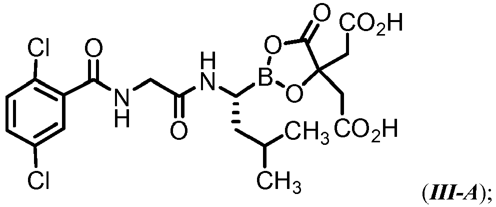 ou l'un des sels pharmaceutiquement acceptables ou l'une des compositions pharmaceutiques de celui-ci ;
où le cancer est choisi dans le groupe constitué par les suivants : cancer de l'ovaire, cancer de l'estomac, cancer du nasopharynx, cancer du poumon à cellules squameuses, mélanome et cancer colorectal.

2. Méthode de détermination s'il faut traiter un patient atteint de cancer avec un inhibiteur de protéasome ou un sel pharmaceutiquement acceptable ou une composition pharmaceutique de celui-ci ;
comprenant les étapes consistant à :
a) mesurer le niveau de NFκB p65 dans un échantillon tumoral issu du patient sous forme de score H, où le score H est déterminé par un dosage IHC de NFκB p65 ; et
b) déterminer s'il faut traiter le patient par une quantité thérapeutiquement active de l'inhibiteur de protéasome ou de l'un des sels pharmaceutiquement acceptables ou l'une des compositions pharmaceutiques de celui-ci, si l'échantillon tumoral est **caractérisé en ce qu'**il présente un score H compris entre 250 et 300, comme mesuré par un dosage IHC de NFκB p65, où :
l'inhibiteur de protéasome est **caractérisé par** le composé de formule ***(III-A)*** : 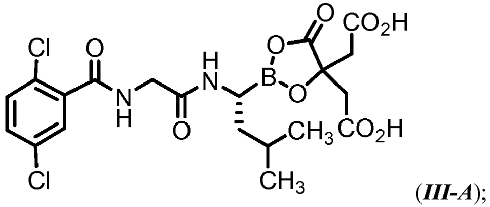 ou l'un des sels pharmaceutiquement acceptables ou l'une des compositions pharmaceutiques de celui-ci ;
où le cancer est choisi dans le groupe constitué par les suivants : cancer de l'ovaire, cancer de l'estomac, cancer du nasopharynx, cancer du poumon à cellules squameuses, mélanome et cancer colorectal.

3. Composé pour utilisation ou méthode selon l'une quelconque des revendications précédentes, où le dosage IHC de NFκB p65 comprend l'utilisation d'un anticorps qui se lie à une protéine avec la séquence de SEQ ID NO: 1.

4. Composé pour utilisation selon la revendication 1 ou méthode selon la revendication 2, où le dosage IHC de NFκB p65 mesure la quantité totale de protéines avec les séquences de SEQ ID NO:1, 2, 3 et 4.

5. Composé pour utilisation ou méthode selon l'une quelconque des revendications précédentes, où le dosage IHC de NFκB p65 mesure la quantité de NFκB p65 présente dans le cytoplasme.

6. Composé pour utilisation ou méthode selon l'une quelconque des revendications précédentes, où le composé de formule ***(III-A)*** est administré par voie orale ou intraveineuse.

7. Composé pour utilisation ou méthode selon l'une quelconque des revendications précédentes, où le composé de formule ***(III-A)*** est administré aux jours 1, 8 et 15 d'un cycle de 28 jours ou aux jours 1, 4, 8 et 11 d'un cycle de 21 jours.

8. Composé pour utilisation ou méthode selon l'une quelconque des revendications précédentes, où le composé de formule ***(III-A)*** est administré par voie orale dans une ou plusieurs capsules.

9. Composé pour utilisation ou méthode selon l'une quelconque des revendications précédentes, où la quantité du composé de formule ***(III-A)*** est comprise entre 2,3 mg et 5,5 mg par dose en se basant sur la quantité du composé de formule ***(II)** ;*


### SEQUENCE LISTING

<110> Di Bacco, Alessandra M.
   Blakemore, Stephen J.
   Mulligan, George J.
   Millennium Pharmaceuticals, Inc.
<120> METHODS OF TREATMENT OF CANCER
<130> MPI12-005P1RNWOM
<150> 61/590131
   <151> 2012-01-24
<160> 4
<170> FastSEQ for Windows Version 4.0
<210> 1
   <211> 551
   <212> PRT
   <213> Homo sapiens
<400> 1
<210> 2
   <211> 548
   <212> PRT
   <213> Homo sapiens
<400> 2
   Met Asp Glu Leu Phe Pro Leu Ile Phe Pro Ala Glu Pro Ala Gln Ala
    1               5                  10                  15
   Ser Gly Pro Tyr Val Glu Ile Ile Glu Gln Pro Lys Gln Arg Gly Met
               20                  25                  30
   Arg Phe Arg Tyr Lys Cys Glu Gly Arg Ser Ala Gly Ser Ile Pro Gly
           35                  40                  45
   Glu Arg Ser Thr Asp Thr Thr Lys Thr His Pro Thr Ile Lys Ile Asn
       50                  55                  60
   Gly Tyr Thr Gly Pro Gly Thr Val Arg Ile Ser Leu Val Thr Lys Asp
   65                  70                  75                  80
   Pro Pro His Arg Pro His Pro His Glu Leu Val Gly Lys Asp Cys Arg
                   85                  90                  95
   Asp Gly Phe Tyr Glu Ala Glu Leu Cys Pro Asp Arg Cys Ile His Ser
               100                 105                 110
   Phe Gln Asn Leu Gly Ile Gln Cys Val Lys Lys Arg Asp Leu Glu Gln
           115                 120                 125
   Ala Ile Ser Gln Arg Ile Gln Thr Asn Asn Asn Pro Phe Gln Glu Glu
       130                 135                 140
   Gln Arg Gly Asp Tyr Asp Leu Asn Ala Val Arg Leu Cys Phe Gln Val
   145                 150                 155                 160
   Thr Val Arg Asp Pro Ser Gly Arg Pro Leu Arg Leu Pro Pro Val Leu
                   165                 170                 175
   Ser His Pro Ile Phe Asp Asn Arg Ala Pro Asn Thr Ala Glu Leu Lys
                   180                     185                     190
   Ile Cys Arg Val Asn Arg Asn Ser Gly Ser Cys Leu Gly Gly Asp Glu
           195                     200                     205
   Ile Phe Leu Leu Cys Asp Lys Val Gln Lys Glu Asp Ile Glu Val Tyr
       210                     215                     220
   Phe Thr Gly Pro Gly Trp Glu Ala Arg Gly Ser Phe Ser Gln Ala Asp
   225                     230                     235                     240
   Val His Arg Gln Val Ala Ile Val Phe Arg Thr Pro Pro Tyr Ala Asp
                   245                     250                     255
   Pro Ser Leu Gln Ala Pro Val Arg Val Ser Met Gln Leu Arg Arg Pro
               260                     265                     270
   Ser Asp Arg Glu Leu Ser Glu Pro Met Glu Phe Gln Tyr Leu Pro Asp
           275                     280                     285
   Thr Asp Asp Arg His Arg Ile Glu Glu Lys Arg Lys Arg Thr Tyr Glu
       290                     295                     300
   Thr Phe Lys Ser Ile Met Lys Lys Ser Pro Phe Ser Gly Pro Thr Asp
   305                     310                     315                     320
   Pro Arg Pro Pro Pro Arg Arg Ile Ala Val Pro Ser Arg Ser Ser Ala
                   325                     330                     335
   Ser Val Pro Lys Pro Ala Pro Gln Pro Tyr Pro Phe Thr Ser Ser Leu
               340                     345                     350
   Ser Thr Ile Asn Tyr Asp Glu Phe Pro Thr Met Val Phe Pro Ser Gly
           355                     360                     365
   Gln Ile Ser Gln Ala Ser Ala Leu Ala Pro Ala Pro Pro Gln Val Leu
       370                     375                     380
   Pro Gln Ala Pro Ala Pro Ala Pro Ala Pro Ala Met Val Ser Ala Leu
   385                     390                     395                     400
   Ala Gln Ala Pro Ala Pro Val Pro Val Leu Ala Pro Gly Pro Pro Gln
                   405                     410                     415
   Ala Val Ala Pro Pro Ala Pro Lys Pro Thr Gln Ala Gly Glu Gly Thr
               420                     425                     430
   Leu Ser Glu Ala Leu Leu Gln Leu Gln Phe Asp Asp Glu Asp Leu Gly
           435                     440                     445
   Ala Leu Leu Gly Asn Ser Thr Asp Pro Ala Val Phe Thr Asp Leu Ala
       450                     455                     460
   Ser Val Asp Asn Ser Glu Phe Gln Gln Leu Leu Asn Gln Gly Ile Pro
   465                     470                     475                     480
   Val Ala Pro His Thr Thr Glu Pro Met Leu Met Glu Tyr Pro Glu Ala
                   485                     490                     495
   Ile Thr Arg Leu Val Thr Gly Ala Gln Arg Pro Pro Asp Pro Ala Pro
               500                     505                     510
   Ala Pro Leu Gly Ala Pro Gly Leu Pro Asn Gly Leu Leu Ser Gly Asp
           515                     520                     525
   Glu Asp Phe Ser Ser Ile Ala Asp Met Asp Phe Ser Ala Leu Leu Ser
       530                     535                     540
   Gln Ile Ser Ser
   545
<210> 3
   <211> 482
   <212> PRT
   <213> Homo sapiens
<400> 3
   Met Asp Glu Leu Phe Pro Leu Ile Phe Pro Ala Glu Pro Ala Gln Ala
    1               5                  10                  15
   Ser Gly Pro Tyr Val Glu Ile Ile Glu Gln Pro Lys Gln Arg Gly Met
               20                  25                  30
   Arg Phe Arg Tyr Lys Cys Glu Gly Arg Ser Ala Gly Ser Ile Pro Gly
           35                  40                  45
   Glu Arg Ser Thr Asp Thr Thr Lys Thr His Pro Thr Ile Lys Ile Asn
       50                  55                  60
   Gly Tyr Thr Gly Pro Gly Thr Val Arg Ile Ser Leu Val Thr Lys Asp
   65                  70                  75                  80
   Pro Pro His Arg Pro His Pro His Glu Leu Val Gly Lys Asp Cys Arg
                   85                  90                  95
   Asp Gly Phe Tyr Glu Ala Glu Leu Cys Pro Asp Arg Cys Ile His Ser
               100                 105                 110
   Phe Gln Asn Leu Gly Ile Gln Cys Val Lys Lys Arg Asp Leu Glu Gln
           115                 120                 125
   Ala Ile Ser Gln Arg Ile Gln Thr Asn Asn Asn Pro Phe Gln Val Pro
       130                 135                 140
   Ile Glu Glu Gln Arg Gly Asp Tyr Asp Leu Asn Ala Val Arg Leu Cys
   145                 150                 155                 160
   Phe Gln Val Thr Val Arg Asp Pro Ser Gly Arg Pro Leu Arg Leu Pro
                   165                 170                 175
   Pro Val Leu Ser His Pro Ile Phe Asp Asn Arg Ala Pro Asn Thr Ala
               180                 185                 190
   Glu Leu Lys Ile Cys Arg Val Asn Arg Asn Ser Gly Ser Cys Leu Gly
           195                 200                 205
   Gly Asp Glu Ile Phe Leu Leu Cys Asp Lys Val Gln Lys Glu Asp Ile
       210                 215                 220
   Glu Val Tyr Phe Thr Gly Pro Gly Trp Glu Ala Arg Gly Ser Phe Ser
   225                 230                 235                 240
   Gln Ala Asp Val His Arg Gln Val Ala Ile Val Phe Arg Thr Pro Pro
                   245                 250                 255
   Tyr Ala Asp Pro Ser Leu Gln Ala Pro Val Arg Val Ser Met Gln Leu
               260                 265                 270
   Arg Arg Pro Ser Asp Arg Glu Leu Ser Glu Pro Met Glu Phe Gln Tyr
           275                 280                 285
   Leu Pro Asp Thr Asp Asp Arg His Arg Ile Glu Glu Lys Arg Lys Arg
       290                 295                 300
   Thr Tyr Glu Thr Phe Lys Ser Ile Met Lys Lys Ser Pro Phe Ser Gly
   305                 310                 315                 320
   Pro Thr Asp Pro Arg Pro Pro Pro Arg Arg Ile Ala Val Pro Ser Arg
                   325                 330                 335
   Ser Ser Ala Ser Val Pro Lys Pro Ala Pro Gly Pro Pro Gln Ala Val
               340                 345                 350
   Ala Pro Pro Ala Pro Lys Pro Thr Gln Ala Gly Glu Gly Thr Leu Ser
           355                 360                 365
   Glu Ala Leu Leu Gln Leu Gln Phe Asp Asp Glu Asp Leu Gly Ala Leu
       370                 375                 380
   Leu Gly Asn Ser Thr Asp Pro Ala Val Phe Thr Asp Leu Ala Ser Val
   385                 390                 395                 400
   Asp Asn Ser Glu Phe Gln Gln Leu Leu Asn Gln Gly Ile Pro Val Ala
                   405                 410                 415
   Pro His Thr Thr Glu Pro Met Leu Met Glu Tyr Pro Glu Ala Ile Thr
               420                 425                 430
   Arg Leu Val Thr Gly Ala Gln Arg Pro Pro Asp Pro Ala Pro Ala Pro
           435                 440                 445
   Leu Gly Ala Pro Gly Leu Pro Asn Gly Leu Leu Ser Gly Asp Glu Asp
       450                 455                 460
   Phe Ser Ser Ile Ala Asp Met Asp Phe Ser Ala Leu Leu Ser Gln Ile
   465                 470                 475                 480
   Ser Ser
<210> 4
   <211> 448
   <212> PRT
   <213> Homo sapiens
<400> 4
   Met Asp Glu Leu Phe Pro Leu Ile Phe Pro Ala Glu Pro Ala Gln Ala
    1               5                  10                  15
   Ser Gly Pro Tyr Val Glu Ile Ile Glu Gln Pro Lys Gln Arg Gly Met
               20                  25                  30
   Arg Phe Arg Tyr Lys Cys Glu Gly Arg Ser Ala Gly Ser Ile Pro Gly
           35                  40                  45
   Glu Arg Ser Thr Asp Thr Thr Lys Thr His Pro Thr Ile Lys Ile Asn
       50                  55                  60
   Gly Tyr Thr Gly Pro Gly Thr Val Arg Ile Ser Leu Val Thr Lys Asp
   65                  70                  75                  80
   Pro Pro His Arg Pro His Pro His Glu Leu Val Gly Lys Asp Cys Arg
                   85                  90                  95
   Asp Gly Phe Tyr Glu Ala Glu Leu Cys Pro Asp Arg Cys Ile His Ser
               100                 105                 110
   Phe Gln Asn Leu Gly Ile Gln Cys Val Lys Lys Arg Asp Leu Glu Gln
           115                 120                 125
   Ala Ile Ser Gln Arg Ile Gln Thr Asn Asn Asn Pro Phe Gln Val Pro
       130                 135                 140
   Ile Glu Glu Gln Arg Gly Asp Tyr Asp Leu Asn Ala Val Arg Leu Cys
   145                 150                 155                 160
   Phe Gln Val Thr Val Arg Asp Pro Ser Gly Arg Pro Leu Arg Leu Pro
                   165                 170                 175
   Pro Val Leu Ser His Pro Ile Phe Asp Asn Arg Ala Pro Asn Thr Ala
               180                 185                 190
   Glu Leu Lys Ile Cys Arg Val Asn Arg Asn Ser Gly Ser Cys Leu Gly
           195                 200                 205
   Gly Asp Glu Ile Phe Leu Leu Cys Asp Lys Val Gln Lys Glu Asp Ile
       210                 215                 220
   Glu Val Tyr Phe Thr Gly Pro Gly Trp Glu Ala Arg Gly Ser Phe Ser
   225                 230                 235                 240
   Gln Ala Asp Val His Arg Gln Val Ala Ile Val Phe Arg Thr Pro Pro
                   245                 250                 255
   Tyr Ala Asp Pro Ser Leu Gln Ala Pro Val Arg Val Ser Met Gln Leu
               260                 265                 270
   Arg Arg Pro Ser Asp Arg Glu Leu Ser Glu Pro Met Glu Phe Gln Tyr
           275                 280                 285
   Leu Pro Asp Thr Asp Asp Arg His Arg Ile Glu Glu Lys Arg Lys Arg
       290                 295                 300
   Thr Tyr Glu Thr Phe Lys Ser Ile Met Lys Lys Ser Pro Phe Ser Gly
   305                 310                 315                 320
   Pro Thr Asp Pro Arg Pro Pro Pro Arg Arg Ile Ala Val Pro Ser Arg
                   325                 330                 335
   Ser Ser Ala Ser Val Pro Lys Pro Ala Pro Gln Pro Tyr Pro Phe Thr
               340                 345                 350
   Ser Ser Leu Ser Thr Ile Asn Tyr Asp Glu Phe Pro Thr Met Val Phe
           355                 360                 365
   Pro Ser Gly Gln Ile Ser Gln Ala Ser Ala Leu Ala Pro Ala Pro Pro
       370                 375                 380
   Gln Val Leu Pro Gln Ala Pro Ala Pro Ala Pro Ala Pro Ala Met Val
   385                 390                 395                 400
   Ser Ala Leu Ala Gln Arg Pro Pro Asp Pro Ala Pro Ala Pro Leu Gly
                   405                 410                 415
   Ala Pro Gly Leu Pro Asn Gly Leu Leu Ser Gly Asp Glu Asp Phe Ser
               420                 425                 430
   Ser Ile Ala Asp Met Asp Phe Ser Ala Leu Leu Ser Gln Ile Ser Ser
           435                 440                 445